# EUROPEAN PATENT APPLICATION

(11) **EP 4 477 747 A2**
(43) Date of publication of application: **18.12.2024**
(21) Application number: 24181372.4
(22) Date of filing: 20.08.2020
(51) Int. Cl.: C12N 5/0783

(54) **LENTIVIRAL TRANSDUCTION METHODS**

(30) Priority: 20.08.2019 GB 201911954
(62) Divisional of application: 20761197.1
(71) Applicant: Adaptimmune Limited, Abingdon, Oxfordshire OX14 4RX (GB)
(72) Inventor: SILK, Jonathan, Abingdon OX14 4RX (GB); MCEWEN-SMITH, Rosanna, Abingdon OX14 4RX (GB); JAPELJ, Nika, Abingdon OX14 4RX (GB); HAMILTON, Garth, Abingdon OX14 4RX (GB)
(74) Representative: Mewburn Ellis LLP

(57) **Abstract**

This invention relates to methods of transducing mammalian cells that comprise exposing a population of mammalian cells to a poloxamer in the absence of a lentiviral vector for 6 hours or more to produce a transduction-primed mammalian cell population, exposing the transduction-primed mammalian cell population to a lentiviral vector, such that the T cells are transduced with the lentiviral vector; and then separating the transduced mammalian cells from the poloxamer. Suitable lentiviral vectors may comprise heterologous nucleic acid that encodes an antigen receptor, such as a T Cell Receptor (TCR) or chimeric antigen receptor. This may be useful, for example, in the transduction of T cells or progenitor cells that differentiate into T cells.

## Description

### Field

The present invention relates to the lentiviral transduction of mammalian cells, in particular T cells and progenitors thereof, for example for use in adoptive cellular therapy (ACT).

### Background

T cells (or T lymphocytes) are found widely distributed within tissues and the tumour environment. T cells are distinguished from other lymphocytes by the presence of T cell receptors (TCRs) on the cell surface. The TCR is a multi-subunit transmembrane complex that mediates the antigen-specific activation of T cells. The TCR confers antigen specificity on the T cell, by recognising an antigen peptide ligand that is presented on the target cell by a major histocompatibility complex (MHC) molecule.

Although peptides derived from altered or mutated proteins in tumour target cells can be recognised as foreign by T cells expressing specific TCRs, many antigens on tumour cells are simply upregulated or overexpressed (so called self-antigens) and do not induce a functional T cell response. Therefore, studies have focussed on identifying target tumour antigens which are expressed, or highly expressed, in the malignant but not the normal cell type. Examples of such targets include the cancer/testis (CT) antigen NY-ESO-1, which is expressed in a wide array of human cancers but shows restricted expression in normal tissues (Chen Y-T et al. Proc Natl Acad Sci USA. 1997; 94(5):1914-1918), and the MAGE-A family of CT antigens which are expressed in a very limited number of healthy tissues (Scanlan M. J. et al. Immunol Rev. 2002; 188:22-32).

Identification of such antigens has promoted the development of targeted T cell-based immunotherapy, which has the potential to provide specific and effective cancer therapy (Ho, W.Y. et al. Cancer Cell 2003; 3:1318-1328; Morris, E.C. et al. Clin. Exp. Immunol. 2003; 131:1-7; Rosenberg, S.A. Nature 2001; 411:380-384; Boon, T. and van der Bruggen P. J. Exp. Med. 1996; 183:725-729).

Dishart et al (2003) J Mol Cell Cardiol 35 (2003) 739-748 reports that the presence of poloxamer P-407 increases the lentiviral transduction of endothelial and smooth muscle cells. WO2013/127964 and Hõfig et al J Gene Med 2012 14 549-560 report that poloxamers of 12.8-15kDa, such as synperonic F108, improve cellular transduction with lentiviral vectors.

Robust and efficient transduction methods are required to transduce T cells and progenitor cells thereof with expression vectors encoding receptors, including T cell receptors (TCRs) and chimeric antigen receptors (CARs), which recognise tumour antigens. These methods would be useful for example in providing T cells for use in adoptive T cell therapy, in particular cancer therapy.

### Summary

The present inventors have recognised that the effectiveness of poloxamers in increasing the efficiency of transduction may be improved by exposing mammalian cells to a poloxamer for 6 hours or more before the cells are exposed to the lentiviral vector. This may be useful, for example, in the transduction of T cells or progenitor cells that differentiate into T cells.

An aspect of the invention provides a method of transducing mammalian cells comprising:
(i) exposing a population of mammalian cells to a poloxamer in the absence of a lentiviral vector for 6 hours or more to produce a transduction-primed mammalian cell population and
(ii) exposing the transduction-primed mammalian cell population to a lentiviral vector, such that the T cells are transduced with the lentiviral vector; and
(iii) separating the transduced mammalian cells from the poloxamer.

The lentiviral vector may comprise heterologous nucleic acid encoding an antigen receptor, such as a T Cell Receptor (TCR) or chimeric antigen receptor.

Preferably, the population of mammalian cells is exposed to the poloxamer in the absence of the lentiviral vector for about 1 day.

Preferably, the transduced mammalian cells are separated from the poloxamer after about 3 days exposure to the lentiviral vector.

In some embodiments, a method of transducing mammalian cells may comprise:
(i) exposing a population of mammalian cells on day 0 to a poloxamer in the absence of a lentiviral vector to produce a transduction-primed T cell population and
(ii) exposing the transduction-primed mammalian cell population on day 1 to a lentiviral vector, such that the T cells are transduced with the lentiviral vector; and
(iii) separating the transduced mammalian cells on day 4 from the poloxamer.

Preferably, the mammalian cells are T cells or cells that are capable of differentiating into T cells, such as induced pluripotent stem cells (iPSCs), mesoderm cells (MCs), haemogenic endothelial cells (HECs), haematopoietic stem cells (HPCs) and progenitor T cells.

Other aspects and embodiments of the invention are described in more detail below.

### Brief Description of the Figures

Figure 1 shows the transduction of T-cells by lentivirus encoding MAGE-A10, NY-ESO-1 and MAGE-A4 TCRs in presence of 0.25mg/ml, 1mg/ml or 4mg/ml poloxamer.
Figure 2 shows the number of transduced T-cells at day 7 following transduction at day 1 by lentivirus in presence or absence of 1 mg/ml poloxamer (F108; LentiBOOST^{™}) with or without cell washing on day 4.
Figure 3 shows the efficiency of lentiviral transduction of T cells following transduction at day 1 by lentivirus with 1mg/ml poloxamer (F108; LentiBOOST^{™}) added at day 0, day 1 - 6 hours (day 0 + 18 hours) and day 1.

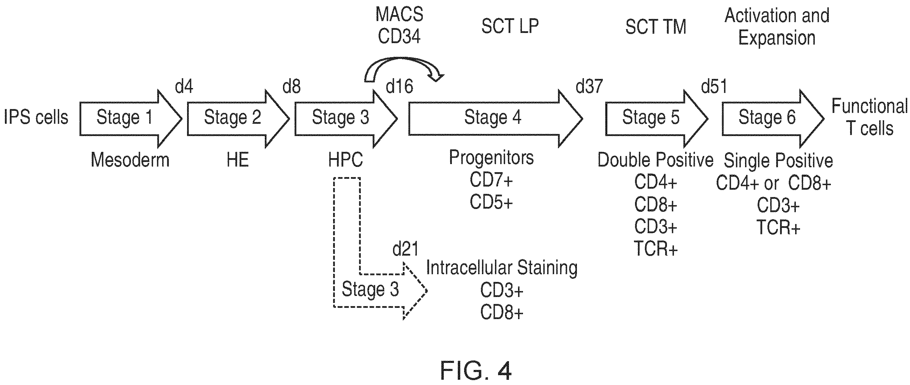

Figure 4 shows a schematic view of an example of a six-stage method for generating T cells from iPSCs.
Figure 5 shows the expression of a TCR in T-cells differentiated from iPSCs where the gene encoding the TCR was introduced by the poloxamer-enhanced transduction of VSV-G pseudotyped lentivirus containing the gene.
Figure 6 shows the increase in cell surface LDL-R caused by incubation of the T-cells in varying concentrations of the poloxamer F108.

### Detailed Description

This invention relates to the *in vitro* transduction of mammalian cells using a lentiviral vector. The mammalian cells are transduction primed through exposure to a poloxamer before being exposed to the lentiviral vector. Pre-priming the mammalian cells with the poloxamer for six hours or longer before exposure to the lentivirus is shown herein to unexpectedly increase the efficiency of lentiviral transduction.

Preferably, the mammalian cells are human cells.

In some embodiments, the mammalian cells may be T cells.

In other embodiments, the mammalian cells may be progenitor cells, for example undifferentiated or partially differentiated cells, that are capable of differentiating into T cells. Progenitor cells may include induced pluripotent stem cells (iPSCs), mesoderm cells (MCs), haemogenic endothelial cells (HECs), haematopoietic stem cells (HPCs) or progenitor T cells (proT cells).

The mammalian cells are transduction primed by exposure to poloxamer. This exposure improves the efficiency of the transduction when primed mammalian cells are contacted with lentiviral vector.

A poloxamer (CAS No. 9003-11-6) is a non-ionic triblock copolymer of ethylene oxide and propylene oxide. A poloxamer is composed of a central hydrophobic chain of polyoxypropylene flanked by two hydrophilic chains of polyoxyethylene (i.e. a polyoxyethylene-polyoxypropylene co-polymer or polyethylene-oxide-polypropylene-oxide co-polymer).

A poloxamer may have the formula (1):

HO-(CH₂-CH₂-O)ₓ-(CH₂-CH(CH₃)-O)_{y}-(CH₂-CH₂-O)ₓ-H,

wherein x and y are independently integers from about 10 to 200 or higher. For example, x may be from about 60 to about 150 and y may be from about 25 to about 60.

Suitable poloxamers include a poloxamer of formula 1 with x having a value of about 101 and y having a value of about 56 (P 407); a poloxamer of formula 1 with x having a value of about 141 and y having a value of about 44 (P 338); a poloxamer of formula 1 with x having a value of about 64 and y having a value of about 37 (P 237); a poloxamer of formula 1 with x having a value of about 80 and y having a value of about 27 (P 188); and a poloxamer of formula 1 with x having a value of about 12 and y having a value of about 20 (P 124). A poloxamer may comprise heterogeneous polymer species of varying chain lengths and the above x and y values may be the average values of all the species that are present.

The nomenclature of poloxamers relates to the approximate molecular weight and percentage of polyoxyethylene content. These values refer to an average value in a poloxamer, rather than an absolute value of each individual poloxamer molecule. The first two digits of a poloxamer number, multiplied by 100, gives the approximate molecular weight of the hydrophobic polyoxypropylene block. The last digit, multiplied by 10, gives the approximate weight percent of the hydrophilic polyoxyethylene content. For example, poloxamer 407 describes a polymer containing a polyoxypropylene hydrophobe of about 4000 Da with the hydrophilic polyoxyethylene content being about 70% of the total molecular weight.

Poloxamers are generally synthesized in two steps, first by building the polyoxypropylene core, and then by addition of polyoxyethylene to the terminal ends of the polyoxypropylene core. Because of variation in the rates of polymerization during both steps, a poloxamer may contain heterogeneous polymer species of varying overall chain lengths and molecular weights. The distribution of polymer species can be characterized using standard techniques including, but not limited to, gel permeation chromatography (GPC).

As a consequence of the technical inability to produce poloxamers in which all molecular species have identical composition, molecular weights and other features of a poloxamer may be described in terms of the average for molecular species of the poloxamer. Average molecular weight as described herein may be number average molecular weight or weight average molecular weight. Suitable methods for determining number average molecular weight and weight average molecular weight are well-known in the art.

A suitable poloxamer may have an average molecular weight of from about 6 to about 18 KDa. For example, about 10 kDa to about 15 kDa, for example about 10 kDa to about 12.6 kDa or about 12.6 kDa to about 15 kDa. Different poloxamers having an average molecular weight of about 10 kDa to about 15 kDa can be generated by changing the length of the polymer blocks making up a poloxamer. In some preferred embodiments, the poloxamer may be poloxamer 407 having an average molecular weight in the range 9840-14600 kDa, for example 12600 to 13600 kDa.

Suitable poloxamers are readily available in the art or may be synthesised using standard techniques. Poloxamers may be known by the trade names of "Pluronics^{™}", "Synperonics^{™}" Flocor^{™}, Lutrol^{®} or Kolliphor^{™}. For example, poloxamer 124 may have the trade names Kollisolv P124 or Lutrol L 44, poloxamer 188 may have the trade names Kolliphor P188 or Lutrol F 68; poloxamer 237 may have the trade names Kolliphor P237 or Lutrol F87; poloxamer 338 may have the trade names Kolliphor P338 or Lutrol F108 (also available as Lentiboost^{™}); poloxamer 407 may have the trade names Kolliphor P407 or Lutrol F127. Other suitable poloxamers may have the trade names Synperonic^{®} L122; Synperonic^{®} P85; Pluronic^{®} F68 and Pluronic^{®} F127 and may be obtained from commercial suppliers (e.g. BASF or Sigma-Aldrich).

Typically, the poloxamer may be dissolved in water to make a stock solution at 100mg/ml and was then sterilised using a 0.2µm filter before storing at 4°C before adding to the priming medium.

The mammalian cell population may be exposed to the poloxamer by culturing the mammalian cells in a priming medium comprising the poloxamer. The priming medium may for example comprise 10 µg/ml to 100mg/ml of the poloxamer. For example, 0.1mg/ml to 10 mg/ml or 0.2mg/ml to 5mg/ml, preferably about 1mg/ml.

The priming medium does not comprise lentiviral vector i.e. the mammalian cell population is primed in the absence of lentivirus.

Any cell culture medium that supports the culture of T cells or progenitors thereof, such as iPSCs, MCs, HECs, HPCs or proT cells, may be supplemented with poloxamer for use as a priming medium. Numerous culture media suitable for use are available, in particular complete media, such as AIM-V, Iscoves medium and RPMI-1640 (Invitrogen-GIBCO). The medium may be supplemented with other factors such as serum, serum proteins and selective agents. For example, in some embodiments, RPMI-1640 medium containing 2 mM glutamine, 10% FBS, 25 mM HEPES, pH 7.2, 1% penicillin-streptomycin, and 55 µM β-mercaptoethanol and optionally supplemented with 20 ng/ml recombinant IL-2 may be employed. Another suitable medium may comprise IMDM medium (Gibco) supplemented with 10% foetal bovine serum (FBS), 1% penicillin and streptomycin, 1% L-glutamine and IL-2. The culture medium may be supplemented with the agonistic or antagonist factors described above at standard concentrations which may readily be determined by the skilled person by routine experimentation.

Conveniently, cells are cultured at 37°C in a humidified atmosphere containing 5% CO₂ in a suitable culture medium.

Methods and techniques for the culture of T cells and other mammalian cells are well-known in the art (see, for example, Basic Cell Culture Protocols, C. Helgason, Humana Press Inc. U.S. (15 Oct 2004) ISBN: 1588295451; Human Cell Culture Protocols (Methods in Molecular Medicine S.) Humana Press Inc., U.S. (9 Dec 2004) ISBN: 1588292223; Culture of Animal Cells: A Manual of Basic Technique, R. Freshney, John Wiley & Sons Inc (2 Aug 2005) ISBN: 0471453293, Ho WY et al J Immunol Methods. (2006) 310:40-52).

The mammalian cells may be cultured in the priming medium comprising the poloxamer for 6 hours or more, 12 hours or more before exposure to the lentiviral vector, for example, 24 hours or more, 36 hours or more or 48 hours or more. Suitable culture conditions for T cells are well-known in the art.

Following exposure to the poloxamer, the transduction primed mammalian cells may be transduced by exposure to a lentiviral vector. The transduction primed mammalian cell population may be exposed to the lentiviral vector by culturing the mammalian cells in a transduction medium comprising the lentiviral vector.

In some preferred embodiments, the poloxamer may be present in the transduction medium. For example, the priming medium may be supplemented with lentiviral vector to produce the transduction medium. In other embodiments, the poloxamer may be absent from the transduction medium.

Lentiviruses are a subtype of retrovirus that are capable of infecting both non-dividing and actively dividing cell types, and include HIV-1, HIV-2, SIV and pSIVgml.

A lentiviral vector is an infectious lentiviral particle that contains heterologous nucleic acid to be expressed in a target cell. For example, a lentiviral vector as described herein may comprise a heterologous nucleic acid encoding an antigen receptor, for example an antigen receptor that binds specifically to cancer cells. The transduction of T cells or progenitors thereof with the lentiviral vector may be useful in generating T cells expressing the antigen receptor for use in therapy.

For safety reasons, lentiviral vectors do not generally contain the genes required for replication. Suitable lentiviral vectors may be conveniently generated according to standard techniques by transfecting a packaging cell line, such as HEK293, with a transfer vector plasmid and two or more helper plasmids. The transfer plasmid contains the heterologous nucleic acid encoding the antigen receptor, flanked by long terminal repeat (LTR) sequences, which facilitate integration of the transfer plasmid sequences into the host cell.The two or more helper plasmids may include a packaging plasmid which encodes virion proteins, such as GAG, POL, TAT, and REV; and an envelope plasmid, which encodes an envelope protein ENV, such as VSV-G; In some embodiments, two packaging plasmids may be employed, a first encoding GAG and POL and a second encoding REV. Following transfection with the transfer plasmid and helper plasmids, the packaging cell line generates infectious lentiviral particles that comprise the nucleic acid encoding the antigen receptor. In some embodiments, a VSVg-pseudotyped viral vector may be produced in combination with the viral envelope glycoprotein G of the Vesicular stomatitis virus (VSVg) to produce a pseudotyped virus particle. For example, solid-phase transduction may be performed without selection by culture on retronectin-coated, retroviral vector-preloaded tissue culture plates.

Lenitiviral particles may be harvested from the cell supernatant and stored and/or concentrated ready for use in transfecting T cells as described herein. Many known techniques and protocols for manipulation and transformation of nucleic acid, for example in preparation of nucleic acid constructs, introduction of DNA into cells and gene expression are described in detail in Protocols in Molecular Biology, Second Edition, Ausubel et al. eds. John Wiley & Sons, 1992. Reagents for generating lentiviral vectors are available from commercial suppliers (e.g. Dharmacon). Suitable techniques for preparing lentiviral vectors are well-known in the art (see for example, Dull, T., et al (1998). J. Virol. 72, 8463-8471; Merten et al (2016) Mol Ther Methods Clin Dev. 2016; 3: 16017).

In some embodiments, the antigen receptor encoded by the heterologous nucleic acid in the lentiviral vector may be a T cell receptor (TCR).

TCRs are disulphide-linked membrane anchored heterodimeric proteins, typically comprising highly variable alpha (α) and beta (β) chains expressed as a complex with invariant CD3 chain molecules. T cells expressing these type of TCRs are referred to as α:β (or αβ) T cells. A minority of T cells express an alternative TCR comprising variable gamma (γ) and delta (δ) chains and are referred to as γδ T cells. TCRs bind specifically to major histocompatibility complexes (MHC) on the surface of cells that display a peptide fragment of a target antigen. For example, TCRs may bind specifically to a major histocompatibility complex (MHC) on the surface of cancer cells that displays a peptide fragment of a tumour antigen. An MHC is a set of cell-surface proteins which allow the acquired immune system to recognise 'foreign' molecules. Proteins are intracellularly degraded and presented on the surface of cells by the MHC. MHCs displaying 'foreign' peptides, such a viral or cancer associated peptides, are recognised by T cells with the appropriate TCRs, prompting cell destruction pathways. MHCs on the surface of cancer cells may display peptide fragments of tumour antigen i.e. an antigen which is present on a cancer cell but not the corresponding non-cancerous cell. T cells which recognise these peptide fragments may exert a cytotoxic effect on the cancer cell.

Preferably, the TCR is not naturally expressed by the T cells (i.e. the TCR is exogenous or heterologous).

Heterologous TCRs may include αβTCR heterodimers and γδTCR heterodimers. Suitable heterologous TCR may bind specifically to class I or II MHC molecules displaying peptide fragments of a target antigen. For example, the T cells may be modified to express a heterologous TCR that binds specifically to class I or II MHC molecules displaying peptide fragments of a tumour antigen expressed by the cancer cells in a cancer patient. Tumour antigens expressed by cancer cells in the cancer patient may identified using standard techniques. Preferred tumour antigens include NY-ESO1, PRAME, alpha-fetoprotein (AFP), MAGE A4, MAGE A1, MAGE A10 and MAGE B2, most preferably NY-ESO-1, MAGE-A4 and MAGE-A10.

Heterologous TCRs may also include unconventional TCRs, for example non-MHC dependent TCRs that bind recognize non-peptide antigens displayed by monomorphic antigen-presenting molecules, such as CD1 and MR1; NKT cell TCRs and intraepithelial lymphocyte (IEL) TCRs.

A heterologous TCR may be a synthetic or artificial TCR i.e. a TCR that does not exist in nature. For example, a heterologous TCR may be engineered to increase its affinity or avidity for a tumour antigen (i.e. an affinity enhanced TCR). The affinity enhanced TCR may comprise one or more mutations relative to a naturally occurring TCR, for example, one or more mutations in the hypervariable complementarity determining regions (CDRs) of the variable regions of the TCR α and β chains or γ and δ chains. These mutations increase the affinity of the TCR for MHCs that display a peptide fragment of a tumour antigen expressed by cancer cells. Suitable methods of generating affinity enhanced TCRs include screening libraries of TCR mutants using phage or yeast display and are well known in the art (see for example Robbins et al J Immunol (2008) 180(9):6116; San Miguel et al (2015) Cancer Cell 28 (3) 281-283; Schmitt et al (2013) Blood 122 348-256; Jiang et al (2015) Cancer Discovery 5 901). Preferred affinity enhanced TCRs may bind to cancer cells expressing one or more of the tumour antigens NY-ESO1, PRAME, alpha-fetoprotein (AFP), MAGE A4, MAGE A1, MAGE A10 and MAGE B2.

Nucleic acid encoding the antigen receptor may encode all the sub-units of the receptor. For example, nucleic acid encoding a TCR may comprise a nucleotide sequence encoding a TCR α chain and a nucleotide sequence encoding a TCR β chain or a nucleotide sequence encoding a TCR γ chain and a nucleotide sequence encoding a TCR δ chain. Suitable nucleotide sequences are well known in the art.

Preferred affinity enhanced TCRs may bind to cancer cells expressing one or more of the tumour antigens NY-ESO1, PRAME, alpha-fetoprotein (AFP), MAGE A4, MAGE A1, MAGE A10 and MAGE B2.

Alternatively, the antigen receptor encoded by the heterologous nucleic acid in the lentiviral vector may be a chimeric antigen receptor (CAR). CARs are artificial receptors that are engineered to contain an immunoglobulin antigen binding domain, such as a single-chain variable fragment (scFv). A CAR may, for example, comprise an scFv fused to a TCR CD3 transmembrane region and endodomain. An scFv is a fusion protein of the variable regions of the heavy (V_{H}) and light (V_{L}) chains of immunoglobulins, which may be connected with a short linker peptide of approximately 10 to 25 amino acids (Huston J.S. et al. Proc Natl Acad Sci USA 1988; 85(16):5879-5883). The linker may be glycine-rich for flexibility, and serine or threonine rich for solubility, and may connect the N-terminus of the V_{H} to the C-terminus of the V_{L}, or vice versa. The scFv may be preceded by a signal peptide to direct the protein to the endoplasmic reticulum, and subsequently the T cell surface. In the CAR, the scFv may be fused to a TCR transmembrane and endodomain. A flexible spacer may be included between the scFv and the TCR transmembrane domain to allow for variable orientation and antigen binding. The endodomain is the functional signal-transmitting domain of the receptor. An endodomain of a CAR may comprise, for example, intracellular signalling domains from the CD3 ζ-chain, or from receptors such as CD28, 41BB, or ICOS. A CAR may comprise multiple signalling domains, for example, but not limited to, CD3z-CD28-41BB or CD3z-CD28-OX40.

The CAR may bind specifically to a tumour-specific antigen expressed by cancer cells. For example, the T cells may be modified to express a CAR that binds specifically to a tumour antigen that is expressed by the cancer cells in a specific cancer patient. Tumour antigens expressed by cancer cells in the cancer patient may identified using standard techniques.

Alternatively, the antigen receptor encoded by the heterologous nucleic acid in the lentiviral vector may be an NK cell receptor (NKCR).

Expression of a heterologous antigen receptor, such as a heterologous TCR, NKCR or CAR may alter the immunogenic specificity of T cells produced as described herein so that they recognise or display improved recognition for one or more target antigens, e.g. tumour antigens that are present on the surface of the cancer cells of an individual with cancer. In some embodiments, the T cells produced as described herein may display reduced binding or no binding to cancer cells in the absence of the heterologous antigen receptor. For example, expression of the heterologous TCR may increase the affinity and/or specificity of the cancer cell binding of a T cell relative to T cells that do not express the antigen receptor.

The term "heterologous" refers to a polypeptide or nucleic acid that is foreign to a particular biological system, such as a host cell or virus, and is not naturally present in that system. A heterologous polypeptide or nucleic acid may be introduced to a biological system by artificial means, for example using recombinant techniques. For example, heterologous nucleic acid encoding a polypeptide may be inserted into a suitable expression construct which is in turn used to transform a host cell to produce the polypeptide. A heterologous polypeptide or nucleic acid may be synthetic or artificial or may exist in a different biological system, such as a different species or cell type. An endogenous polypeptide or nucleic acid is native to a particular biological system, such as a host cell, and is naturally present in that system. A recombinant polypeptide is expressed from heterologous nucleic acid that has been introduced into a cell by artificial means, for example using recombinant techniques. A recombinant polypeptide may be identical to a polypeptide that is naturally present in the cell or may be different from the polypeptides that are naturally present in that cell.

The T cells or progenitors thereof are transduced to express a heterologous antigen receptor which specifically binds to target cells from a patient, for example cancer cells of a cancer patient, using a lentiviral vector as described herein. The cancer patient may be subsequently treated with the T cells. Suitable cancer patients for treatment with the T cells may be identified by a method comprising;
obtaining sample of cancer cells from an individual with cancer and;
identifying the cancer cells as binding to the antigen receptor expressed by the T cells.

Cancer cells may be identified as binding to the antigen receptor by identifying one or more tumour antigens expressed by the cancer cells. Methods of identifying antigens on the surface of cancer cells obtained from an individual with cancer are well-known in the art.

In some embodiments, a heterologous antigen receptor suitable for the treatment of a specific cancer patient may be identified by;
obtaining sample of cancer cells from an individual with cancer and;
identifying an antigen receptor that specifically binds to the cancer cells.

An antigen receptor that specifically binds to the cancer cells may be identified for example by identifying one or more tumour antigens expressed by the cancer cells. Methods of identifying antigens on the surface of cancer cells obtained from an individual with cancer are well-known in the art. An antigen receptor which binds to the one or more tumour antigens or which binds to MHC-displayed peptide fragments of the one or more antigens may then be identified, for example from antigen receptors of known specificities or by screening a panel or library of antigen receptors with diverse specificities. Antigen receptors that specifically bind to cancer cells having one or more defined tumour antigens may be produced using routine techniques.

The T cells or progenitors thereof may be transduced with a lentiviral vector that encodes the identified antigen receptor as described herein.

The cancer cells of an individual suitable for treatment as described herein may express the antigen and may be of correct HLA type to bind the antigen receptor.

Cancer cells may be distinguished from normal somatic cells in an individual by the expression of one or more antigens (i.e. tumour antigens). Normal somatic cells in an individual may not express the one or more antigens or may express them in a different manner, for example at lower levels, in different tissue and/or at a different developmental stage. Tumour antigens may elicit immune responses in the individual. In particular, a tumour antigen may elicit a T cell-mediated immune response against cancer cells in the individual that express the tumour antigen. One or more tumour antigens expressed by cancer cells in a patient may be selected as a target antigen for heterologous receptors on modified T cells.

Tumour antigens expressed by cancer cells may include, for example, cancer-testis (CT) antigens encoded by cancer-germ line genes, such as MAGE-A1, MAGE-A2, MAGE- A3, MAGE-A4, MAGE-A5, MAGE-A6, MAGE-A7, MAGE-A8, MAGE-A9, MAGE-A10, MAGE-A11, MAGE-A12, GAGE-I, GAGE-2, GAGE-3, GAGE-4, GAGE-5, GAGE-6, GAGE-7, GAGE-8, BAGE-I, RAGE- 1, LB33/MUM-1, PRAME, NAG, MAGE-Xp2 (MAGE-B2), MAGE-Xp3 (MAGE-B3), MAGE-Xp4 (MAGE-B4), MAGE- C1/CT7, MAGE-C2, NY-ESO-I, LAGE-I, SSX-I, SSX-2(HOM-MEL-40), SSX-3, SSX-4, SSX-5, SCP-I and XAGE and immunogenic fragments thereof (Simpson et al. Nature Rev (2005) 5, 615-625, Gure et al., Clin Cancer Res (2005) 11, 8055-8062; Velazquez et al., Cancer Immun (2007) 7, 1 1 ; Andrade et al., Cancer Immun (2008) 8, 2; Tinguely et al., Cancer Science (2008); Napoletano et al., Am J of Obstet Gyn (2008) 198, 99 e91-97).

Other tumour antigens include, for example, overexpressed, upregulated or mutated proteins and differentiation antigens particularly melanocyte differentiation antigens such as p53, ras, CEA, MUC1, PMSA, PSA, tyrosinase, Melan-A, MART-1, gp100, gp75, alpha-actinin-4, Bcr-Abl fusion protein, Casp-8, beta-catenin, cdc27, cdk4, cdkn2a, coa-1, dek-can fusion protein, EF2, ETV6-AML1 fusion protein, LDLR-fucosyltransferaseAS fusion protein, HLA-A2, HLA-A11, hsp70-2, KIAAO205, Mart2, Mum-2, and 3, neo-PAP, myosin class I, OS-9, pml-RAR.alpha. fusion protein, PTPRK, K-ras, N-ras, triosephosphate isomerase, GnTV, Herv-K-mel, NA-88, SP17, and TRP2-Int2, (MART-I), E2A-PRL, H4-RET, IGH-IGK, MYL-RAR, Epstein Barr virus antigens, EBNA, human papillomavirus (HPV) antigens E6 and E7, TSP-180, MAGE-4, MAGE-5, MAGE-6, p185erbB2, p180erbB-3, c-met, nm-23H1, PSA, TAG-72-4, CA 19-9, CA 72-4, CAM 17.1, NuMa, K-ras, alpha.-fetoprotein, 13HCG, BCA225, BTAA, CA 125, CA 15-3 (CA 27.29\BCAA), CA 195, CA 242, CA-50, CAM43, CD68\KP1, CO-029, FGF-5, G250, Ga733 (EpCAM), HTgp-175, M344, MA-50, MG7-Ag, MOV18, NB\170K, NY-CO-1, RCAS1, SDCCAG16, TA-90 (Mac-2 binding protein\cyclophilin C-associated protein), TAAL6, TAG72, TLP, TPS and tyrosinase related proteins such as TRP-1, TRP-2.

Other tumour antigens include out-of-frame peptide-MHC complexes generated by the non-AUG translation initiation mechanisms employed by "stressed" cancer cells (Malarkannan et al. Immunity 1999 Jun; 10(6):681-90).

Other tumour antigens are well-known in the art (see for example WO00/20581; Cancer Vaccines and Immunotherapy (2000) Eds Stern, Beverley and Carroll, Cambridge University Press, Cambridge) The sequences of these tumour antigens are readily available from public databases but are also found in WO 1992/020356 A1, WO 1994/005304 A1, WO 1994/023031 A1, WO 1995/020974 A1, WO 1995/023874 A1 and WO 1996/026214 A1.

Preferred tumour antigens include NY-ESO1, PRAME, alpha-fetoprotein (AFP), MAGE A4, MAGE A1, MAGE A10 and MAGE B2, most preferably NY-ESO-1 and MAGE-A10.

NY-ESO-1 is a human tumour antigen of the cancer/testis (CT) family and is frequently expressed in a wide variety of cancers, including melanoma, prostate, transitional cell bladder, breast, lung, thyroid, gastric, head and neck, and cervical carcinoma (van Rhee F. et al. Blood 2005; 105(10): 3939-3944). In addition, expression of NY-ESO-1 is usually limited to germ cells and is not expressed in somatic cells (Scanlan M.J. et al. Cancer Immun. 2004; 4(1)). Suitable affinity enhanced TCRs that bind to cancer cells expressing NY-ESO-1 include NY-ESO-1^{c²⁵⁹}.

NY-ESO-1 c²⁵⁹ is an affinity enhanced TCR is mutated at positions 95 and 96 of the alpha chain 95:96LY relative to the wild-type TCR. NY-ESO-1 c²⁵⁹ binds to a peptide corresponding to amino acid residues 157-165 of the human cancer testis Ag NY-ESO-1 (SLLMWITQC) in the context of the HLA-A2+ class 1 allele with increased affinity relative to the unmodified wild type TCR (Robbins et al J Immunol (2008) 180(9):6116).

MAGE-A10 is a highly immunogenic member of the MAGE-A family of CT antigens, and is expressed in germ cells but not in healthy tissue. MAGE-A10 is expressed in high percentages of cancer cells from a number of tumours (Schultz-Thater E. et al. Int J Cancer. 2011; 129(5):1137-1148).

The mammalian cell population may be exposed to the lentiviral vector by culturing the mammalian cells in a transduction medium comprising the lentiviral vector. Suitable methods of lentiviral transduction are well known in the art. For example, packaged lentiviral vector may be introduced to the mammalian cells and the mammalian cells incubated for 3 days or more.

The mammalian cells may be cultured at any convenient cell density typically between 0.5×10⁶ and 1×10⁶ cells/ml.

The multiplicity of infection (MOI) of the transduction may be 0.1 to 1000 lentiviral vectors per cell preferably 1 to 100.

Preferably, the transduced cells are washed after 2-4 days, for example 3 days, exposure to the lentiviral vector and the poloxamer (i.e. on day 3-5, preferably day 4). Suitable washing techniques are well known in the art.

Following transduction and optional washing, the transduced mammalian cell population may be isolated and/or purified, for example using fluorescence-activated cell sorting (FACS) and antibody coated magnetic particles. The expression of the heterologous antigen receptor by the mammalian cells may be determined.

The transduced mammalian cells may be cultured *in vitro* such that the cells proliferate and expand the population. For example, a transduced T cell population may be expanded using magnetic beads coated with anti-CD3 and anti-CD28. The transduced mammalian cells may be cultured using any convenient technique to produce the expanded population. Suitable culture systems include stirred tank fermenters, airlift fermenters, roller bottles, culture bags or dishes, and other bioreactors, in particular hollow fibre bioreactors. The use of such systems is well-known in the art.

Optionally, the population of mammalian cells transduced as described herein may be stored, for example by cryopreservation, before use.

In some preferred embodiments, the mammalian cells for transduction as described herein are T cells.

T cells (also called T lymphocytes) are white blood cells that play a central role in cell-mediated immunity. T cells can be distinguished from other lymphocytes by the presence of a T cell receptor (TCR) on the cell surface. There are several types of T cells, each type having a distinct function. T helper cells (T_{H} cells) are known as CD4⁺ T cells because they express the CD4 surface glycoprotein. CD4⁺ T cells play an important role in the adaptive immune system and help the activity of other immune cells by releasing T cell cytokines and helping to suppress or regulate immune responses. They are essential for the activation and growth of cytotoxic T cells. Cytotoxic T cells (Tc cells, CTLs, killer T cells) are known as CD8⁺ T cells because they express the CD8 surface glycoprotein. CD8⁺ T cells act to destroy virus-infected cells and tumour cells. Most CD8⁺ T cells express TCRs that can recognise a specific antigen displayed on the surface of infected or damaged cells by a class I MHC molecule. Specific binding of the TCR and CD8 glycoprotein to the antigen and MHC molecule leads to T cell-mediated destruction of the infected or damaged cells.

T cells for transduction as described herein may be single positive CD4⁺ T cells; single positive CD8⁺ T cells; and/or double positive CD4⁺ CD8⁺ T cells. For example, the T cells may be a mixed population of CD4⁺ T cells and CD8⁺ T cells.

T cells for transduction as described herein may be mature CD3+ T cells. For example, the cells may have a TCR+ CD3+ phenotype. In some embodiments, T cells may also express CD45 and CD28.

In some embodiments, T cells for transduction as described herein may be obtained from a donor individual. A method described herein may comprise the step of obtaining T cells from a donor individual and/or isolating T cells from a sample obtained from a donor individual. In other embodiments, T cells previously obtained from a donor individual or previously isolated from a sample obtained from a donor individual may be employed. The donor individual may be a healthy individual or an individual with cancer.

The donor individual may be the same person as the recipient individual to whom the T cells will be administered following modification and expansion as described herein (autologous treatment). Alternatively, the donor individual may be a different person to the recipient individual to whom the T cells will be administered following modification and expansion as described herein (allogeneic treatment). For example, the donor individual may be a healthy individual who is human leukocyte antigen (HLA) matched (either before or after donation) with a recipient individual suffering from cancer.

A population of T cells may be isolated from a blood sample obtained from the donor individual. Suitable methods for the isolation of T cells and other peripheral blood cells are well-known in the art and include, for example fluorescent activated cell sorting (FACS: see for example, Rheinherz et al (1979) PNAS 76 4061), cell panning (see for example, Lum et al (1982) Cell Immunol 72 122) and isolation using antibody coated magnetic beads (see, for example, Gaudernack et al 1986 J Immunol Methods 90 179).

CD4⁺ and CD8⁺ T cells may be isolated from the population of peripheral blood mononuclear cells (PBMCs) obtained from a blood sample. PBMCs may be extracted from a blood sample using standard techniques. For example, ficoll may be used in combination with gradient centrifugation (Böyum A. Scand J Clin Lab Invest. 1968; 21(Suppl.97):77-89), to separate whole blood into a top layer of plasma, followed by a layer of PBMCs and a bottom fraction of polymorphonuclear cells and erythrocytes. In some embodiments, the PBMCs may be depleted of CD14⁺ cells (monocytes).

Following isolation, T cells may be activated. Suitable methods for activating T cells are well known in the art. For example, the isolated T cells may be exposed to a T cell receptor (TCR) agonist. Suitable methods for activating and expanding T cells are well-known in the art. For example, T cells may be exposed to a T cell receptor (TCR) agonist under appropriate culture conditions. Suitable TCR agonists include ligands, such as peptides displayed on a class I or II MHC molecule (MHC-peptide complexes) on the surface of a bead or an antigen presenting cell, such as a dendritic cell, and soluble factors, such as anti-TCR antibodies for example antibody CD28 antibodies, and multimeric MHC-peptide complexes, such as MHC-peptide tetramers, pentamers or dextramers..

Activation refers to the state of a T cell that has been sufficiently stimulated to induce detectable cellular proliferation. Activation can also be associated with induced cytokine production, and detectable effector functions. The term "activated T cells" refers to, among other things, T cells that are undergoing cell division.

An anti-TCR antibody may specifically bind to a component of the TCR, such as εCD3, αCD3 or αCD28. Anti-TCR antibodies suitable for TCR stimulation are well-known in the art (e.g. OKT3) and available from commercial suppliers (e.g. eBioscience CO USA). In some embodiments, T cells may be activated by exposure to anti-αCD3 antibodies and IL2, IL7 or IL15. More preferably, T cells are activated by exposure to anti-αCD3 antibodies and anti-αCD28 antibodies. The activation may occur in the presence or absence of CD14⁺ monocytes. The T cells may be activated with anti-CD3 and anti-CD28 antibody coated beads. For example, PBMCs or T cell subsets including CD4⁺ and/or CD8⁺ cells may be activated, without feeder cells (antigen presenting cells) or antigen, using antibody coated beads, for example magnetic beads coated with anti-CD3 and anti-CD28 antibodies, such as Dynabeads^{®} Human T-Activator CD3/CD28 (ThermoFisher Scientific). In other embodiments, soluble tetrameric antibody complexes that bind CD3, CD28 and CD2 cell surface ligands, such as ImmunoCult^{™} Human CD3/CD28/CD2 T Cell Activator or Human CD3/CD28 T Cell Activator, may be used to activate the T cells. In other embodiments, T cells may be activated with an MHC-peptide complex, preferably a multimeric MHC-peptide complex, optionally in combination with an anti-CD28 antibody. T cells expressing a chimeric antigen receptor may be activated using a soluble antigen to the receptor. The antigen may be in a multimeric form or on the surface of a bead and may optionally be used in conjunction with an anti-TCR antibody, such as an anti-CD28 antibody.

Following isolation and/or activation, the T cells obtained from the donor individual may be primed for lentiviral transduction using a poloxamer as described herein.

In other embodiments, T cells for transduction as described herein may be generated from a population of iPSCs.

iPSCs are pluripotent cells which are derived from non-pluripotent, fully differentiated donor or antecedent cells. iPSCs are capable of self-renewal *in vitro* and exhibit an undifferentiated phenotype and are potentially capable of differentiating into any foetal or adult cell type of any of the three germ layers (endoderm, mesoderm and endoderm). The population of iPSCs may be clonal i.e. genetically identical cells descended from a single common ancestor cell. iPSCs may express one or more of the following pluripotency associated markers: POU5f1 (Oct4), Sox2, Alkaline Phosphatase, SSEA-3, Nanog, SSEA-4, Tra-1-60, KLF4 and c-myc, preferably one or more of POU5f1, NANOG and SOX2. An iPSC may lack markers associated with specific differentiative fates, such as Bra, Sox17, FoxA2, αFP, Sox1, NCAM, GATA6, GATA4, Hand1 and CDX2. In particular, an iPSC may lack markers associated with endodermal fates.

Preferably, the iPSCs are human IPSCs (hiPSCs).

In some embodiments, iPSCs may be gene edited, for example to inactivate or delete HLA genes or other genes associated with immunogenicity or GVHD.

iPSCs may be derived or reprogramed from donor cells, which may be somatic cells or other antecedent cells obtained from a source, such as a donor individual. The donor cells may be mammalian, preferably human cells. Suitable donor cells include adult fibroblasts and blood cells, for example peripheral blood cells, such as HPCs or mononuclear cells.

Suitable donor cells for reprogramming into iPSCs as described herein may be obtained from a donor individual. In some embodiments, the donor individual may be the same person as the recipient individual to whom the T cells will be administered following production as described herein (autologous treatment). In other embodiments, the donor individual may be a different person to the recipient individual to whom the T cells will be administered following production as described herein (allogeneic treatment). For example, the donor individual may be a healthy individual who is human leukocyte antigen (HLA) matched (either before or after donation) with a recipient individual suffering from cancer. In other embodiments, the donor individual may not be HLA matched with the recipient individual. Preferably, the donor individual may be a neonate (new-born), for example the donor cells may be obtained from a sample of umbilical cord blood.

Suitable donor individuals are preferably free of communicable viral (e.g. HIV, HPV, CMV) and adventitious agents (e.g. bacteria, mycoplasma), and free of known genetic abnormalities.

In some embodiments, a population of peripheral blood cells, such as haematopoietic progenitor cells (HPCs), for reprogramming may be isolated from a blood sample, preferably an umbilical cord sample, obtained from the donor individual as described above. HPCs may be identified in a sample of blood cells by expression of CD34. In other embodiments, a population of fibroblasts for reprogramming may be isolated from a skin biopsy following dispersal using collagenase or trypsin and out-growth in appropriate cell culture conditions.

In some embodiments, IPSCs may be derived from antigen-specific T cells. For example, the T cells may comprise nucleic acid encoding αβ TCRs that bind to an antigen, such as a tumor antigen, displayed in complex with a class I MHC. Antigen-specific T cells for use in the generation of iPSCs may be obtained by screening a diverse population of T cells with peptide epitopes from the target antigen displayed on a class I or II MHC molecule on the surface of an antigen presenting cell, such as a dendritic cell, or by isolating from a tumour sample from a cancer patient.

Donor cells are typically reprogrammed into iPSCs by the introduction of reprogramming factors, such as Oct4, Sox2 and Klf4 into the cell. The reprogramming factors may be proteins or encoding nucleic acids and may be introduced into the differentiated cells by any suitable technique, including plasmid, transposon or more preferably, viral transfection or direct protein delivery. Other reprogramming factors, for example Klf genes, such as Klf-1, -2, -4 and -5; Myc genes such as C-myc, L-myc and N-myc; Nanog; SV40 Large T antigen; Lin28; and short hairpins (shRNA) targeting genes such as p53, may also be introduced into the cell to increase induction efficiency. Following introduction of the reprogramming factors, the donor cells may be cultured. Cells expressing pluripotency markers may be isolated and/or purified to produce a population of iPSCs. Techniques for the production of iPSCs are well-known in the art (Yamanaka et al Nature 2007; 448:313-7; Yamanaka 6 2007 Jun 7; 1(1):39-49; Kim et al Nature. 2008 Jul 31; 454(7204):646-50; Takahashi Cell. 2007 Nov 30; 131(5):861-72. Park et al Nature. 2008 Jan 10; 451(7175):141-6; Kimet et al Cell Stem Cell. 2009 Jun 5;4(6):472-6; Vallier, L., et al. Stem Cells, 2009. 9999(999A): p. N/A; Baghbaderani et al 2016; Stem Cell Rev. 2016 Aug; 12(4):394-420; Baghbaderani et al. (2015) Stem Cell Reports, 5(4), 647-659).

Conventional techniques may be employed for the culture and maintenance of iPSCs (Vallier, L. et al Dev. Biol. 275, 403-421 (2004), Cowan, C.A. et al. N. Engl. J. Med. 350, 1353-1356 (2004), Joannides, A. et al. Stem Cells 24, 230-235 (2006) Klimanskaya, I. et al. Lancet 365, 1636-1641 (2005), Ludwig, T.E. et al. Nat. Biotechnol. 24, 185-187 (2006)). iPSCs for use in the present methods may be grown in defined conditions or on feeder cells. For example, iPSCs may be conventionally cultured in a culture dish on a layer of feeder cells, such as irradiated mouse embryonic fibroblasts (MEF), at an appropriate density (e.g. 10⁵ to 10⁶ cells/60mm dish), or on an appropriate substrate, in a feeder conditioned or defined iPSC maintenance medium. iPSCs for use in the present methods may be passaged by enzymatic or mechanical means. In some embodiments, iPSCs may be passaged on matrigel^{™} or an ECM protein, such as vitronectin, in an iPSC maintenance medium, such as mTeSR1 (StemCell Technologies) or E8 flex (Life Thermo) culture medium.

T cells may be produced from a population of iPSCs by a method comprising;
(i) differentiating the population of iPSCs into mesoderm cells (MCs),
(ii) differentiating the MCs to produce a population of haemogenic endothelial cells (HECs),
(iii) differentiating the HECs into a population of haematopoietic progenitor cells (HPCs),
(iv) differentiating the population of HPCs into progenitor T (proT) cells;
(v) maturing the progenitor T cells to produce a population of double positive CD4+ CD8+ T cells, and
(vi) expanding and/or activating the double positive CD4+ CD8+ T cells to produce a population of single positive CD4+ or CD8+ T cells.

The mammalian cells may be transduced with a lentiviral vector using a method described herein at any one of stages (i) to (vi). For example, any one of the (a) iPSCs, (b) MCs (c) HECs (d) HPCs, (e) progenitor T cells, (f) double positive T cells or (g) single positive T cells may be transfected or transduced with a lentiviral vector as described herein.

Differentiation and maturation of the cell populations in the steps of the methods described herein is induced by culturing the cells in a culture medium supplemented with a set of differentiation factors. The set of differentiation factors that is listed for each culture medium is preferably exhaustive and medium may be devoid of other differentiation factors. In preferred embodiments, the culture media are chemically defined media. For example, a culture medium may consist of a chemically defined nutrient medium that is supplemented with an effective amount of one or more differentiation factors, as described below. A chemically defined nutrient medium may comprise a basal medium that is supplemented with one or more serum-free culture medium supplements.

Differentiation factors are factors which modulate, for example promote or inhibit, a signalling pathway which mediates differentiation in a mammalian cell. Differentiation factors may include growth factors, cytokines and small molecules which modulate one or more of the Activin/Nodal, FGF, Wnt or BMP signalling pathways. Examples of differentiation factors include Activin/Nodal, FGFs, BMPs, retinoic acid, vascular endothelial growth factor (VEGF), stem cell factor (SCF), TGFβ ligands, GDFs, LIF, Interleukins, GSK-3 inhibitors and phosphatidylinositol 3-kinase (PI3K) inhibitors.

Differentiation factors which are used in one or more of the media described herein include TGFβ ligands, such as activin, fibroblast growth factor (FGF), bone morphogenetic protein (BMP), stem cell factor (SCF), vascular endothelial growth factor (VEGF), GSK-3 inhibitors (such as CHIR-99021), interleukins, and hormones, such as IGF-1 and angiotensin II. A differentiation factor may be present in a medium described herein in an amount that is effective to modulate a signalling pathway in cells cultured in the medium.

In some embodiments, a differentiation factor listed above or below may be replaced in a culture medium by a factor that has the same effect (i.e. stimulation or inhibition) on the same signalling pathway. Suitable factors are known in the art and include proteins, nucleic acids, antibodies and small molecules.

The extent of differentiation of the cell population during each step may be determined by monitoring and/or detecting the expression of one or more cell markers in the population of differentiating cells. For example, an increase in the expression of markers characteristic of the more differentiated cell type or a decrease in the expression of markers characteristic of the less differentiated cell type may be determined. The expression of cell markers may be determined by any suitable technique, including immunocytochemistry, immunofluorescence, RT-PCR, immunoblotting, fluorescence activated cell sorting (FACS), and enzymatic analysis. In preferred embodiments, a cell may be said to express a marker if the marker is detectable on the cell surface. For example, a cell which is stated herein not to express a marker may display active transcription and intracellular expression of the marker gene but detectable levels of the marker may not be present on the surface of the cell.

A population of partially differentiated cells that is produced by a step in the methods described herein may be cultured, maintained or expanded before the next differentiation step. Partially differentiated cells may be expanded by any convenient technique.

After each step, the population of partially differentiated cells which is produced by that step may contain 1% or more, 5% or more, 10% or more or 15% or more partially differentiated cells, following culture in the medium. If required, the population of partially differentiated cells may be purified by any convenient technique, such as MACs or FACS.

Cells may be cultured in a monolayer, in the absence of feeder cells, on a surface or substrate coated with extracellular matrix protein, such as fibronectin, laminin or collagen. Suitable techniques for cell culture are well-known in the art (see, for example, Basic Cell Culture Protocols, C. Helgason, Humana Press Inc. U.S. (15 Oct 2004) ISBN: 1588295451; Human Cell Culture Protocols (Methods in Molecular Medicine S.) Humana Press Inc., U.S. (9 Dec 2004) ISBN: 1588292223; Culture of Animal Cells: A Manual of Basic Technique, R. Freshney, John Wiley & Sons Inc (2 Aug 2005) ISBN: 0471453293, Ho WY et al J Immunol Methods. (2006) 310:40-52, Handbook of Stem Cells (ed. R. Lanza) ISBN: 0124366430) Basic Cell Culture Protocols' by J. Pollard and J. M. Walker (1997), 'Mammalian Cell Culture: Essential Techniques' by A. Doyle and J. B. Griffiths (1997), 'Human Embryonic Stem Cells' by A. Chiu and M. Rao (2003), Stem Cells: From Bench to Bedside' by A. Bongso (2005), Peterson & Loring (2012)Human Stem Cell Manual: A Laboratory Guide Academic Press and 'Human Embryonic Stem Cell Protocols' by K. Turksen (2006). Media and ingredients thereof may be obtained from commercial sources (e.g. Gibco, Roche, Sigma, Europa bioproducts, R&D Systems). Standard mammalian cell culture conditions may be employed for the above culture steps, for example 37°C, 5% or 21% Oxygen, 5% Carbon Dioxide. Media is preferably changed every two days and cells allowed to settle by gravity.

Cells may be cultured in a culture vessel. Suitable cell culture vessels are well-known in the art and include culture plates, dishes, flasks, bioreactors, and multi-well plates, for example 6-well, 12-well or 96-well plates.

The culture vessels are preferably treated for tissue culture, for example by coating one or more surfaces of the vessel with an extracellular matrix protein, such as fibronectin, laminin or collagen. Culture vessels may be treated for tissue culture using standard techniques, for example by incubating with a coating solution, as described herein, or may be obtained pre-treated from commercial suppliers.

In a first stage, iPSCs may be differentiated into mesoderm cells by culturing the population of iPSCs under suitable conditions to promote mesodermal differentiation. For example, the iPSCs cells may be cultured sequentially in first, second and third mesoderm induction media to induce differentiation into mesoderm cells.

A suitable first mesoderm induction medium may stimulate SMAD2 and SMAD3 mediated signalling pathways. For example, the first mesoderm induction medium may comprise activin.

A suitable second mesoderm induction medium may (i) stimulate SMAD1, SMAD2, SMAD3, SMAD5 and SMAD9 mediated signalling pathways and (ii) have fibroblast growth factor (FGF) activity. For example, the second mesoderm induction medium may comprise activin, preferably activin A, BMP, preferably BMP4 and FGF, preferably bFGF.

A suitable third mesoderm induction medium may (i) stimulate SMAD1, SMAD2, SMAD3, SMAD5 and SMAD9 mediated signalling pathways (ii) have fibroblast growth factor (FGF) activity and (iii) inhibit glycogen synthase kinase 3β. For example, the third mesoderm induction medium may comprise activin, preferably activin A, BMP, preferably BMP4, FGF, preferably bFGF, and a GSK3 inhibitor, preferably CHIR99021.

The first, second and third mesoderm induction media may be devoid of differentiation factors other than the differentiation factors set out above.

SMAD2 and SMAD3 mediated intracellular signalling pathways may be stimulated by the first, second and third mesoderm induction media through the presence in the media of a first TGFβ ligand. The first TGFβ ligand may be Activin. Activin (Activin A: NCBI Gene ID: 3624 nucleic acid reference sequence NM_002192.2 Gl: 62953137, amino acid reference sequence NP_002183.1 Gl: 4504699) is a dimeric polypeptide which exerts a range of cellular effects via stimulation of the Activin/Nodal pathway (Vallier et al., Cell Science 118:4495-4509 (2005)). Activin is readily available from commercial sources (e.g. Stemgent Inc. MA USA; Miltenyi Biotec Gmbh, DE). Conveniently, the concentration of Activin in a medium described herein may be from 1 to 100ng/ml, preferably about 5 to 50ng/ml.

The fibroblast growth factor (FGF) activity of the second and third mesoderm induction media may be provided by the presence of fibroblast growth factor (FGF) in the media. Fibroblast growth factor (FGF) is a protein factor which stimulates cellular growth, proliferation and cellular differentiation by binding to a fibroblast growth factor receptor (FGFR). Suitable fibroblast growth factors include any member of the FGF family, for example any one of FGF1 to FGF14 and FGF15 to FGF23. Preferably, the FGF is FGF2 (also known as bFGF, NCBI GenelD: 2247, nucleic acid sequence NM_002006.3 Gl: 41352694, amino acid sequence NP_001997.4 Gl: 41352695); FGF7 (also known as keratinocyte growth factor (or KGF), NCBI GenelD: 2247, nucleic acid sequence NM_002006.3 Gl: 41352694, amino acid sequence NP_001997.4 Gl: 41352695); or FGF10 (NCBI GenelD: 2247, nucleic acid sequence NM_002006.3 Gl: 41352694, amino acid sequence NP_001997.4 Gl: 41352695). Most preferably, the fibroblast growth factor is FGF2.

Conveniently, the concentration of FGF, such as FGF2 in a medium described herein may be from 0.5 to 50ng/ml, preferably about 5ng/ml. Fibroblast growth factors, such as FGF2, FGF7 and FGF10, may be produced using routine recombinant techniques or obtained from commercial suppliers (e.g. R&D Systems, Minneapolis, MN; Stemgent Inc, USA; Miltenyi Biotec Gmbh, DE). SMAD1, SMAD5 and SMAD9 mediated intracellular signalling pathways may be stimulated by the second and third mesoderm induction media through the presence in the media of a second TGFβ ligand. The second TGFβ ligand may be a Bone Morphogenic Protein (BMP). Bone Morphogenic Proteins (BMPs) bind to Bone Morphogenic Protein Receptors (BMPRs) and stimulate intracellular signalling through pathways mediated by SMAD1, SMAD5 and SMAD9. Suitable Bone Morphogenic Proteins include any member of the BMP family, for example BMP2, BMP3, BMP4, BMP5, BMP6 or BMP7. Preferably the second TGFβ ligand is BMP2 (NCBI GenelD: 650, nucleic acid sequence NM_001200.2 Gl: 80861484; amino acid sequence NP_001191.1 Gl: 4557369) or BMP4 (NCBI GenelD: 652, nucleic acid sequence NM_001202.3 Gl: 157276592; amino acid sequence NP_001193.2 Gl: 157276593). Suitable BMPs include BMP4. Conveniently, the concentration of a Bone Morphogenic Protein, such as BMP2 or BMP4 in a medium described herein may be from 1 to 500ng/ml, preferably about 10ng/ml. BMPs may be produced using routine recombinant techniques or obtained from commercial suppliers (e.g. R&D, Minneapolis, USA, Stemgent Inc, USA; Miltenyi Biotec Gmbh, DE).

The GSK3β inhibition activity of the third mesoderm induction medium may be provided by the presence of a GSK3β inhibitor in the medium. GSK3β inhibitors inhibit the activity of glycogen synthase kinase 3β (Gene ID 2932: EC2.7.11.26). Preferred inhibitors specifically inhibit the activity of glycogen synthase kinase 3β. Suitable inhibitors include CHIR99021 (*6-((2-((4-(2,4-Dichlorophenyl)-5-(4-methyl-1H-imidazol-2-yl)pyrimidin-2-yl)amino)ethyl)amino)nicotinonitrile;* Ring D. B. et al., Diabetes, 52:588-595 (2003)) alsterpaullone, kenpaullone, BIO(6-bromoindirubin-3'-oxime (Sato et al Nat Med. 2004 Jan;10(1):55-63), SB216763 *(3-(2,4-dichlorophenyl)-4-(1-methyl-1H-indol-3-yl)-1H-pyrrole-2,5-dione),* Lithium and SB415286 *(3-[(3-chloro-4-hydroxyphenyl)amino]-4-(2-nitrophenyl)-1H-pyrrole-2,5-dione;* Coghlan et al Chem Biol. 2000 Oct;7(10):793-803). In some preferred embodiments, the GSK3β inhibitor is CHIR99021.Suitable glycogen synthase kinase 3β inhibitors may be obtained from commercial suppliers (e.g. Stemgent Inc. MA USA; Cayman Chemical Co. MI USA; Selleckchem, MA USA). For example, the third mesoderm induction medium may contain 0.1 to 100µM of a GSK3β inhibitor, such as CHIR99021, preferably about 10µM.

In preferred embodiments, the first, second and third mesoderm induction media are chemically defined media. For example, the first mesoderm induction medium may consist of a chemically defined nutrient medium supplemented with an effective amount of activin, preferably activin A, for example 50ng/ml activin A; the second mesoderm induction medium may consist of a chemically defined nutrient medium supplemented with an effective amount of activin preferably activin A, for example 5ng/ml activin A, BMP, preferably BMP4, for example 10ng/ml BMP4; and FGF, preferably bFGF (FGF2), for example 5ng/ml bFGF; and the third mesoderm induction medium may consist of a chemically defined nutrient medium supplemented with an effective amount of activin preferably activin A, for example 5ng/ml activin A, BMP, preferably BMP4, for example 10ng/ml BMP4; FGF, preferably bFGF (FGF2), for example 5ng/ml bFGF; and GSK3 inhibitor, preferably CHIR-99021 , for example 10µM CHIR-99021.

A chemically defined medium (CDM) is a nutritive solution for culturing cells which contains only specified components, preferably components of known chemical structure. A CDM is devoid of undefined components or constituents which include undefined components, such as feeder cells, stromal cells, serum, and complex extracellular matrices, such as matrigel^{™} For example, a CDM does not contain stromal cells, such as OP9 cells, expressing Notch ligands, such as DLL1 or DLL4.

The chemically defined nutrient medium may comprise a chemically defined basal medium. Suitable chemically defined basal media include Iscove's Modified Dulbecco's Medium (IMDM), Ham's F12, Advanced Dulbecco's modified eagle medium (DMEM) (Price et al Focus (2003), 25 3-6), Williams E (Williams, G.M. et al Exp. Cell Research, 89, 139-142 (1974)), RPMI-1640 (Moore, G.E. and Woods L.K., (1976) Tissue Culture Association Manual. 3, 503-508) and StemPro^{™}-34 PLUS (ThermoFisher Scientific).

The basal medium may be supplemented by serum-free culture medium supplements and/or additional components in the medium. Suitable supplements and additional components are described above and may include L-glutamine or substitutes, such as GlutaMAX-1^{™}, ascorbic acid, monothiolglycerol (MTG), antibiotics such as penicillin and streptomycin, human serum albumin, for example recombinant human serum albumin, such as Cellastim^{™} (Merck/Sigma) and Recombumin^{™} (albumedix.com), insulin, transferrin and 2-mercaptoethanol. A basal medium may be supplemented with a serum substitute, such as Knockout Serum Replacement (KOSR; Invitrogen).

The iPSCs may be cultured in the first mesoderm induction medium for 1 to 12 hours, preferably about 4 hours; then cultured in the second mesoderm induction medium for 30 to 54 hours, preferably about 44 hours; and then cultured in the third mesoderm induction medium for 36 to 60 hours, preferably about 48 hours to produce a population of mesodermal cells.

Mesoderm cells are partially differentiated progenitor cells that are committed to mesodermal lineages and are capable of differentiation under appropriate conditions into all cell types in the mesenchyme (fibroblast), muscle, bone, adipose, vascular and haematopoietic systems. Mesoderm cells may express one or more mesodermal markers. For example, the mesoderm cells may express any one, two, three, four, five, six or all seven of Brachyury, Goosecoid, Mixl1, KDR, FoxA2, GATA6 and PDGFaR.

In a second stage, mesoderm cells may be differentiated into haemogenic endothelial cells (HECs) by culturing the population of mesoderm cells under suitable conditions to promote haemogenic endothelial (HE) differentiation. For example, the iPSCs cells may be cultured in an HE induction medium.

A suitable HE induction medium may (i) stimulate cKIT receptor (CD117) mediated signalling pathways and (ii) stimulate VEGFR mediated signalling pathways. For example, the HE induction medium may comprise SCF and VEGF.

Vascular endothelial growth factor (VEGF) is a protein factor of the PDGF family which binds to VEGFR tyrosine kinase receptors and stimulates vasculogenesis and angiogenesis. Suitable VEGFs include any member of the VEGF family, for example any one of VEGF-A to VEGF-D and PIGF. Preferably, the VEGF is VEGF-A (also known as VEGF, NCBI Gene ID: 7422, nucleic acid sequence NM_001025366.2, amino acid sequence NP_001020537.2). VEGF is readily available from commercial sources (e.g. R&D Systems, USA). Conveniently, the concentration of VEGF in an HE induction medium described herein may be from 1 to 100ng/ml, for example any of about 5, 7, 10, 12, 15, 17, 20, 25, 30, 35, 40, 45 or 50 ng/ml, preferably about 15 ng/ml.

In some examples of HE induction media, VEGF may be replaced by a VEGF activator or agonist that stimulates VEGFR mediated signalling pathways. Suitable VEGF activators are known in the art and include proteins, such as gremlin (Mitola et al (2010) Blood 116(18) 3677-3680) nucleic acids, such as shRNA (e.g. Turunen et al Circ Res. 2009 Sep 11; 105(6):604-9), CRISPR-based plasmids (e.g. VEGF CRISPR activation plasmid; Santa Cruz Biotech, USA), antibodies and small molecules.

Stem cell factor (SCF) is a cytokine that binds to the KIT receptor (KIT proto-oncogene, receptor tyrosine kinase) (CD117; SCFR) and is involved in haematopoiesis. SCF (also called KITLG, NCBI GenelD: 4254) may have the reference nucleic acid sequence NM_000899.5 or NM_03994.5 and the reference amino acid sequence NP_000890.1 or NP_003985.5. SCF is readily available from commercial sources (e.g. R&D Systems, USA). Conveniently, the concentration of SCF in an HE induction medium described herein may be from 1 to 1000ng/ml, for example any of about 10, 20, 30, 40, 50, 60, 70, 80, 90, 100, 110, 120, 130, 140, 150, 200, 250, 300, 350, 400, 450, 500, 600, 700, 800, 900 ng/ml, preferably about 100 ng/ml.

In preferred embodiments, the HE induction medium is a chemically defined medium. For example, the HE induction medium may consist of a chemically defined nutrient medium supplemented with effective amounts of VEGF, for example 15ng/ml VEGF; and SCF, for example 100ng/ml SCF.

Suitable chemically defined nutrient media are described above and include StemPro^{™}-34 (ThermoFisher Scientific).

The mesoderm cells may be cultured in the HE induction medium for 2 to 6 days, preferably about 4 days, to produce a population of HE cells.

Haemogenic endothelial cells (HECs) are partially differentiated endothelial progenitor cells that have hematopoietic potential and are capable of differentiation under appropriate conditions into haematopoietic lineages. HE cells may express CD34. In some embodiments, HECs may not express CD73 or CXCR4 (CD184). For example, the HE cells may have the phenotype CD34+ CD73- or CD34+ CD73- CXCR4-.

In a third stage, haemogenic endothelial (HE) cells may be differentiated into haematopoietic progenitor cells (HPCs) by culturing the population of HE cells under suitable conditions to promote haematopoietic differentiation. For example, the HE cells may be cultured in a haematopoietic induction medium.

A suitable haematopoietic induction medium may stimulate the following (i) cKIT receptor (CD117) mediated signalling pathways, (ii) VEGFR mediated signalling pathways, (iii) MPL (CD110) mediated signalling pathways (iv) FLT3 mediated signalling pathways (v) IGF1R mediated signalling pathways (vi) SMAD1, 5 and 9 mediated signalling pathways (vii) Hedgehog signalling pathways (viii) EpoR mediated signalling pathway and (ix) AGTR2 mediated signalling pathways. A suitable haematopoietic induction medium may also inhibit the AGTR1 (angiotensin II type 1 receptor (AT₁)) mediated signaling pathway. A suitable haematopoietic induction medium may also have interleukin (IL) activity and FGF activity.

For example, a haematopoietic induction medium may comprise the differentiation factors: VEGF, SCF, Thrombopoietin (TPO), Flt3 ligand (Flt3L), IL-3, IL-6, IL-7, IL-11, IGF-1, BMP, FGF, Sonic hedgehog (SHH), erythropoietin (EPO), angiotensin II, and an angiotensin II type 1 receptor (AT₁) antagonist. An example of a suitable haematopoietic induction medium is the Stage 3 medium shown in Table 1 below.

Thrombopoietin (TPO) is a glycoprotein hormone that regulates platelet production. TPO (also called THPO, NCBI Gene ID: 7066) may have the reference nucleic acid sequence NM_000460.4 and the reference amino acid sequence NP_000451.1. TPO is readily available from commercial sources (e.g. R&D Systems, USA; Miltenyi Biotec Gmbh, DE). Conveniently, the concentration of TPO in a haematopoietic induction medium described herein may be from 3 to 300ng/ml, for example any of about 3, 4, 5, 6, 7, 8, 9, 10, 11, 12, 13, 14, 15, 20, 25, 27, 30, 32, 35, 40, 45, 50, 60, 70, 80, 90 or 100, 110, 120, 130, 140, 150, 160, 170, 180, 190, 200, 225, 250, 275 or 300 ng/ml, preferably about 30 ng/ml.

Flt3 ligand (Fms-related tyrosine kinase 3 ligand or FLT3L) is a cytokine with haematopoietic activity which binds to the FLT3 receptor and stimulates the proliferation and differentiation of progenitor cells. Flt3 ligand (also called FLT3LG, NCBI GenelD: 2323) may have the reference nucleic acid sequence NM_001204502.2 and the reference amino acid sequence NP_001191431.1. Flt3 is readily available from commercial sources (e.g. R&D Systems, USA; Miltenyi Biotec Gmbh, DE). Conveniently, the concentration of Flt3 ligand in a haematopoietic induction medium described herein may be from 0.25 to 250ng/ml, for example any of about 0.1, 0.25, 0.5, 1, 2, 3, 4, 5, 6, 7, 8, 9, 10, 11, 12, 13, 14, 15, 20, 25, 30, 35, 40, 45, 50, 60, 70, 80, 90 or 100, 110, 120, 130, 140, 150, 160, 170, 180, 190, 200, 210, 220, 230 or 240 ng/ml, preferably about 25 ng/ml.

Interleukins (ILs) are cytokines that play major roles in immune development and function. ILs in a haematopoietic induction medium may include IL-3, IL-6, IL-7, and IL-11.

IL-3 (also called IL3 or MCGF, NCBI GenelD: 3562) may have the reference nucleic acid sequence NM_000588.4 and the reference amino acid sequence NP_000579.2. IL-3 is readily available from commercial sources (e.g. R&D Systems, USA; Miltenyi Biotec Gmbh, DE). Conveniently, the concentration of IL-3 in a haematopoietic induction medium described herein may be from 0.25 to 250ng/ml, for example any of about 0.1, 0.25, 0.5, 1, 2, 3, 4, 5, 6, 7, 8, 9, 10, 11, 12, 13, 14, 15, 20, 25, 30, 35, 40, 45, 50, 60, 70, 80, 90 or 100, 110, 120, 130, 140, 150, 160, 170, 180, 190, 200, 210, 220, 230 or 240 ng/ml, preferably about 25 ng/ml.

IL-6 (also called IL6 or HGF, NCBI GenelD: 3569) may have the reference nucleic acid sequence NM_000600.5 and the reference amino acid sequence NP_000591.5. IL-6 is readily available from commercial sources (e.g. R&D Systems, USA; Miltenyi Biotec Gmbh, DE). Conveniently, the concentration of IL-6 in a haematopoietic induction medium described herein may be from 0.1 to 100ng/ml, for example any of about 0.1, 0.25, 0.5, 1, 2, 3, 4, 5, 6, 7, 8, 9, 10, 11, 12, 13, 14, 15, 20, 25, 30, 35, 40, 45, 50, 60, 70, 80, 90 or 95 ng/ml, preferably about 10 ng/ml.

IL-7 (also called IL7, NCBI GenelD: 3574) may have the reference nucleic acid sequence NM_000880.4 and the reference amino acid sequence NP_000871.1. IL-7 is readily available from commercial sources (e.g. R&D Systems, USA; Miltenyi Biotec Gmbh, DE). Conveniently, the concentration of IL-7 in a haematopoietic induction medium described herein may be from 0.1 to 100ng/ml, for example any of about 0.1, 0.25, 0.5, 0.75, 1, 2, 3, 4, 5, 6, 7, 8, 9, 10, 11, 12, 13, 14, 15, 20, 25, 30, 35, 40, 45, 50, 60, 70, 80, 90 or 95 ng/ml, preferably about 10 ng/ml.

IL-11 (also called AGIF, NCBI GenelD: 3589) may have the reference nucleic acid sequence NM_000641.4 and the reference amino acid sequence NP_000632.1. IL-11 is readily available from commercial sources (e.g. R&D Systems, USA; Miltenyi Biotec Gmbh, DE). Conveniently, the concentration of IL-11 ligand in a haematopoietic induction medium described herein may be from 0.5 to 100ng/ml, for example any of about 0.1, 0.25, 0.5, 1, 2, 3, 4, 5, 6, 7, 8, 9, 10, 11, 12, 13, 14, 15, 20, 25, 30, 35, 40, 45, 50, 60, 70, 80, 90 or 95 ng/ml, preferably about 5 ng/ml.

Insulin-like growth factor 1 (IGF-1) is a hormone that binds to the tyrosine kinases IGF-1 receptor (IGF1R) and insulin receptor and activates the multiple signalling pathways. IGF-1(also called IGF or MGF, NCBI GenelD: 3479) may have the reference nucleic acid sequence NM_000618.5 and the reference amino acid sequence NP_000609.1. IGF-1 is readily available from commercial sources (e.g. R&D Systems, USA). Conveniently, the concentration of IGF-1 in a haematopoietic induction medium described herein may be from 0.25 to 250ng/ml, for example any of about 0.1, 0.25, 0.5, 1, 2, 3, 4, 5, 6, 7, 8, 9, 10, 11, 12, 13, 14, 15, 20, 25, 30, 35, 40, 45, 50, 60, 70, 80, 90 or 100, 110, 120, 130, 140, 150, 160, 170, 180, 190, 200, 210, 220, 230 or 240 ng/ml, preferably about 25 ng/ml.

Sonic hedgehog (SHH) is a ligand of the hedgehog signalling pathway that regulates vertebrate organogenesis. SHH (also called TPT or HHG1, NCBI GenelD: 6469) may have the reference nucleic acid sequence NM_000193.4 and the reference amino acid sequence NP_000184.1. SHH is readily available from commercial sources (e.g. R&D Systems, USA; Miltenyi Biotec Gmbh, DE). Conveniently, the concentration of SHH in a haematopoietic induction medium described herein may be from 0.25 to 250ng/ml, for example any of about 0.1, 0.25, 0.5, 1, 2, 3, 4, 5, 6, 7, 8, 9, 10, 11, 12, 13, 14, 15, 20, 25, 30, 35, 40, 45, 50, 60, 70, 80, 90 or 100, 110, 120, 130, 140, 150, 160, 170, 180, 190, 200, 210, 220, 230 or 240 ng/ml, preferably about 25 ng/ml.

Erythropoietin (EPO) is a glycoprotein cytokine that binds to the erythropoietin receptor (EpoR) and stimulates erythropoiesis. EPO (also called DBAL, NCBI GenelD: 2056) may have the reference nucleic acid sequence NM_000799.4 and the reference amino acid sequence NP_000790.2. EPO is readily available from commercial sources (e.g. R&D Systems, USA; PreproTech, USA). Conveniently, the concentration of EPO in haematopoietic induction medium described herein may be from 0.02 to 20 U/ml, for example any of about 0.05, 0.1, 0.25, 0.5, 1, 2, 3, 4, 5, 6, 7, 8, 9, 10, 11, 12, 13, 14, 15, or 18 U/ml, preferably about 2U/ml.

Angiotension II is a heptapeptide hormone that is formed by the action of angiotensin converting enzyme (ACE) on angiotensin I. Angiotension II stimulates vasoconstriction. Angiotension I and II are formed by the cleavage of angiotensinogen (also called AGT, NCBI GenelD: 183), which may have the reference nucleic acid sequence NM_000029.4 and the reference amino acid sequence NP_000020.1. Angiotension II is readily available from commercial sources (e.g. R&D Systems, USA; Tocris, USA). Conveniently, the concentration of angiotension II in a haematopoietic induction medium described herein may be from 0.05 to 50ng/ml, for example any of about 0.1, 0.25, 0.5, 1, 2, 3, 4, 5, 6, 7, 8, 9, 10, 11, 12, 13, 14, 15, 20, 25, 30, 35, 40, 45, or 48 ng/ml, preferably about 5 ng/ml.

Angiotensin II type 1 receptor (AT₁) antagonists (ARBs) are compounds that selectively block the activation of AT, receptor (AGTR1; Gene ID 185). Suitable AT₁ antagonists include losartan (2-Butyl-4-chloro-1-{[2'-(1H-tetrazol-5-yl)-4-biphenylyl]methyl}-1H-imidazol-5-yl)methanol), valsartan ((2S)-3-Methyl-2-(pentanoyl{[2'-(1H-tetrazol-5-yl)biphenyl-4-yl]methyl}amino)butanoic acid), and telmisartan (4'[(1,4'-Dimethyl-2'-propyl[2,6'-bi-1H-benzimidazol]-1'-yl)methyl][1,1'-biphenyl]-2-carboxylic acid. In some preferred embodiments, the AT₁ antagonist is losartan. Suitable AT₁ antagonists may be obtained from commercial suppliers (e.g. Tocris, USA; Cayman Chemical Co. MI USA). Conveniently, the concentration of angiotensin II type 1 receptor (AT₁) antagonist in a haematopoietic induction medium described herein may be from 1 to 1000 µM, for example any of about 2, 3, 4, 5, 6, 7, 8, 9, 10, 11, 12, 13, 14, 15, 20, 25, 30, 35, 40, 45, 50, 60, 70, 80, 90 100, 110, 120, 150, 200, 300, 400, 500, 600, 700, 800 or 900 µM, preferably about 100 µM.

In preferred embodiments, the haematopoietic induction medium is a chemically defined medium. For example, the haematopoietic induction medium may consist of a chemically defined nutrient medium supplemented with effective amounts of VEGF, for example 15ng/ml; SCF, for example 100ng/ml; thrombopoietin (TPO), for example 30ng/ml; Flt3 ligand (FLT3L), for example 25ng./ml; IL-3, for example 25ng/ml; IL-6, for example 10ng/ml; IL-7, for example 10 ng/ml; IL-11, for example 5 ng/ml; IGF-1, for example 25 ng/ml; BMP, for example BMP4 at 10ng/ml; FGF, for example bFGF at 5ng/ml; Sonic hedgehog (SHH), for example 25ng/ml; erythropoietin (EPO), for example 2 u/ml; angiotensin II, for example 10µg/ml, and an angiotensin II type 1 receptor (AT₁) antagonist, for example losartan, at 100µM. A suitable haematopoietic induction medium be devoid of other differentiation factors. For example, a haematopoietic induction medium may consist of a chemically defined nutrient medium supplemented with one or more differentiation factors, wherein the one or more differentiation factors consist of VEGF, SCF, Thrombopoietin (TPO), Flt3 ligand (Flt3L), IL-3, IL-6, IL-7, IL-11, IGF-1, BMP, FGF, Sonic hedgehog (SHH), erythropoietin (EPO), angiotensin II, and an angiotensin II type 1 receptor (AT₁) antagonist (i.e. the medium does not contain any differentiation factors other than VEGF, SCF, Thrombopoietin (TPO), Flt3 ligand (Flt3L), IL-3, IL-6, IL-7, IL-11, IGF-1, BMP, FGF, Sonic hedgehog (SHH), erythropoietin (EPO), angiotensin II, and an angiotensin II type 1 receptor (AT₁) antagonist).

Suitable chemically defined nutrient media are described above and include StemPro^{™}-34 PLUS (ThermoFisher Scientific) or a basal medium such as IMDM supplemented with albumin, insulin, selenium transferrin, and lipids as described below.

The HE cells may be cultured in the haematopoietic induction medium for 8-21 days, preferably about 16 days, to produce the population of HPCs.

HPCs (also called hematopoietic stem cells) are multipotent stem cells that are committed to a hematopoietic lineage and are capable of further hematopoietic differentiation into all blood cell types including myeloid and lymphoid lineages, including monocytes, B cells and T cells. HPCs may express CD34. HPCs may co-express CD133, CD45 and FLK1 (also known as KDR or VEGFR2) and may be negative for expression of CD38 and other lineage specific markers. For example, HPCs may display the phenotype CD34+ CD133+ CD45+ FLK1+ CD38-.

Following the generation of HPCs from HE cells, a population of HPCs expressing one or more cell surface markers, such as CD34, may be purified, for example by magnetic activated cell sorting (MACS), before being subjected to further differentiation. For example, a population of CD34+ HPCs may be purified. The CD34+ HPCs may be purified after 8 days, for example 8-10 days, culture in the HE induction medium. The CD34+ HPCs may be purified after 16 days of differentiation, for example on day 16 to day 18 of the differentiation method.

In a fourth stage, haematopoietic progenitor cells (HPCs) may be differentiated into progenitor T cells by culturing the population of HPCs under suitable conditions to promote lymphoid differentiation. For example, the haematopoietic progenitor cells may be cultured in a lymphoid expansion medium.

A lymphoid expansion medium is a cell culture medium that promotes the lymphoid differentiation of HPCs into progenitor T cells.

A suitable lymphoid expansion medium may (i) stimulate cKIT receptor (CD117; KIT receptor tyrosine kinase) mediated signalling pathways, (ii) stimulate MPL (CD110) mediated signalling pathways (iii) FLT3 mediated signalling pathways and (iv) have interleukin (IL) activity. For example, a lymphoid expansion medium may comprise the differentiation factors SCF, FLT3L, TPO and IL7.

In preferred embodiments, the lymphoid expansion medium is a chemically defined medium. For example, the lymphoid expansion medium may consist of a chemically defined nutrient medium supplemented with effective amounts of the above differentiation factors. Suitable lymphoid expansion media are well-known in the art and include Stemspan^{™} SFEM II (Cat # 9605; StemCell Technologies Inc, CA).with Stemspan^{™} lymphoid expansion supplement (Cat # 9915; StemCell Technologies Inc, CA).

The HPCs may be cultured on a surface during differentiation into progenitor T cells. For example, the HPCs may be cultured on a surface of a culture vessel, bead or other biomaterial or polymer.

Preferably, the surface may be coated with a factor that stimulates Notch signalling, for example a Notch ligand, such as Delta-like 1 (DLL1) or Delta-like 4 (DLL4). Suitable Notch ligands are well-known in the art and available from commercial suppliers.

The surface may also be coated with an extracellular matrix protein, such as fibronectin, vitronectin, laminin or collagen and/or one or more cell surface adhesion proteins, such as VCAM1. In some embodiments, the surface for HPC culture may have a coating that comprises a factor that stimulates Notch signalling, for example a Notch ligand, such as DLL4, without the extracellular matrix protein or cell surface adhesion protein.

In some embodiments, the surface for HPC culture may have a coating that comprises a factor that stimulates Notch signalling, for example a Notch ligand, such as DLL4, an extracellular matrix protein, such as vitronectin, and a cell surface adhesion protein, such as VCAM1. The surface may be coated with an extracellular matrix protein, factor that stimulates Notch signalling and cell surface adhesion protein by contacting the surface with a coating solution. For example, the coating solution may be incubated on the surface under suitable conditions to coat the surface. Conditions may, for example, include about 2 hours at room temperature. Coating solutions comprising an extracellular matrix protein and a factor that stimulates Notch signalling are available from commercial suppliers (StemSpan^{™} Lymphoid Differentiation Coating Material; Cat # 9925; Stem Cell Technologies Inc, CA).

The HPCs may be cultured in the lymphoid expansion medium on the substrate for a time sufficient for the HPCs to differentiate into progenitor T cells. For example, the HPCs may be cultured for 2-6 weeks or 2-4 weeks, preferably 3 weeks.

Progenitor T cells are multi-potent lymphopoietic progenitor cells that are capable of giving rise to αβ T cells, γδ T cells, tissue resident T cells and NKT cells. Progenitor T cells may commit to the αβ T cell lineage after pre-TCR selection in the thymus. Progenitor T cells may be capable of in vivo thymus colonization and may be capable of committing to the T cell lineage after pre-TCR selection in the thymus. Progenitor T cells may also be capable of maturation into cytokine-producing CD3⁺ T-cells.

Progenitor T cells may express CD5 and CD7 i.e. the progenitor T cells may have a CD5+CD7+ phenotype. Progenitor T cells may also co-express CD44, CD25 and CD2. For example, progenitor T cells may have a CD5+, CD7+ CD44+, CD25+ CD2+ phenotype. In some embodiments, progenitor T cells may also co-express CD45. Progenitor T cells may lack expression of CD3, CD4 and CD8, for example on the cell surface.

In a fifth stage, progenitor T cells may be matured into T cells by culturing the population of progenitor T cells under suitable conditions to promote T cell maturation. For example, the progenitor T cells may be cultured in a T cell maturation medium.

A T cell maturation medium is a cell culture medium that promotes the maturation of progenitor T cells into mature T cells. A suitable T cell maturation medium may (i) stimulate cKIT receptor (CD117; KIT receptor tyrosine kinase)mediated signalling pathways (ii) FLT3 mediated signalling pathways and (iii) have interleukin (IL) activity. For example, a T cell maturation medium may comprise the differentiation factors SCF, FLT3L, and IL7.

In preferred embodiments, the T cell maturation medium is a chemically defined medium. For example, the T cell maturation medium may consist of a chemically defined nutrient medium supplemented with effective amounts of the above differentiation factors. Suitable T cell maturation media are well-known in the art and include Stemspan^{™} SFEM II (Cat # 9605; StemCell Technologies Inc, CA) with Stemspan^{™} T cell maturation supplement (Cat # 9930; StemCell Technologies Inc, CA) and other media suitable for expansion of PBMCs and CD3+ cells, such as ExCellerate Human T cell expansion medium (R& D Systems, USA). Other suitable T cell maturation media may include a basal medium such as IMDM, supplemented with ITS, albumin and lipids, as described elsewhere herein and further supplemented with effective amounts of the above differentiation factors.

The progenitor T cells may be cultured on a surface. For example, the progenitor T cells may be cultured on a surface of a culture vessel, bead or other biomaterial or polymer.

Preferably, the surface may be coated with a factor that stimulates Notch signalling, for example a Notch ligand, such as Delta-like 1 (DLL1) or Delta-like 4 (DLL4). Suitable Notch ligands are well-known in the art and available from commercial suppliers. The surface may also be coated with an extracellular matrix protein, such as fibronectin, vitronectin, laminin or collagen and/or one or more cell surface adhesion proteins, such as VCAM1. Suitable coatings are well-known in the art and described elsewhere herein.

The progenitor T cells may be cultured in the T cell maturation medium on the substrate for a time sufficient for the progenitor T cells to mature into T cells. For example, the progenitor T cells may be cultured for 1-4 weeks, preferably 2 or 3 weeks.

In some embodiments, the T cells produced by maturation of progenitor T cells may be double positive CD4+CD8+ T cells.

Progenitor T cells may be matured into T cells by the methods described above.

Following maturation of progenitor T cells (stage 5), the population of T cells may be predominantly double positive CD4+CD8+ T cells.

In a sixth stage, the population of double positive T cells may be activated and/or expanded to produce or increase the proportion of single positive CD4+ T cells, or more preferably single positive CD8+ T cells. Suitable methods for activating and expanding T cells are well-known in the art and are described above. In some embodiments, double positive CD4+CD8+ T cells may be cultured in a T cell maturation medium as described herein supplemented with IL-15. The medium may be further supplemented with a T cell receptor (TCR) agonist, for example one or more anti-TCR antibodies, such as anti-αCD3 antibodies, and anti-αCD28 antibodies, as described above in order to activate and expand the population and produce single positive T cells.

Following transduction, T cells produced as described herein may express a heterologous antigen receptor, such as a T cell receptor (TCR) NK cell receptor or chimeric antigen receptor (CAR) that binds a target antigen. For example, the heterologous antigen receptor may bind specifically to cancer cells that express a tumor antigen. The T cells may be useful for example in immunotherapy, as described below.

Following production, the population of T cells, for example DP CD4+CD8+ cells, SP CD4+ cells or SP CD8+ cells, may be isolated and/or purified. Any convenient technique may be used, including fluorescence-activated cell sorting (FACS) or magnetic-activated cell sorting using antibody coated magnetic particles (MACS).

The population of T cells, for example DP CD4+CD8+ cells, SP CD4+ cells or SP CD8+ cells, may be expanded and/or concentrated. Optionally, the population of T cells produced as described herein may be stored, for example by cryopreservation, before use.

A population of T cells transduced as described herein may be admixed with other reagents, such as buffers, carriers, diluents, preservatives and/or pharmaceutically acceptable excipients. Suitable reagents are described in more detail below. A method described herein may comprise admixing the population of modified T cells with a pharmaceutically acceptable excipient.

Pharmaceutical compositions suitable for administration (e.g. by infusion), include aqueous and non-aqueous isotonic, pyrogen-free, sterile injection solutions which may contain anti-oxidants, buffers, preservatives, stabilisers, bacteriostats, and solutes which render the formulation isotonic with the blood of the intended recipient; and aqueous and non-aqueous sterile suspensions which may include suspending agents and thickening agents. Examples of suitable isotonic vehicles for use in such formulations include Sodium Chloride Injection, Ringer's Solution, or Lactated Ringer's Injection. Suitable vehicles can be found in standard pharmaceutical texts, for example, Remington's Pharmaceutical Sciences, 18th edition, Mack Publishing Company, Easton, Pa., 1990.

In some preferred embodiments, the population of T cells transduced as described herein may be formulated into a pharmaceutical composition suitable for intravenous infusion into an individual.

The term "pharmaceutically acceptable" as used herein pertains to compounds, materials, compositions, and/or dosage forms which are, within the scope of sound medical judgement, suitable for use in contact with the tissues of a subject (e.g., human) without excessive toxicity, irritation, allergic response, or other problem or complication, commensurate with a reasonable benefit/risk ratio. Each carrier, excipient, etc. must also be "acceptable" in the sense of being compatible with the other ingredients of the formulation.

A population of T cells transduced as described herein may be for use as a medicament. For example, a population of T cells may be used in cancer immunotherapy therapy, for example adoptive T cell therapy.

Adoptive cellular therapy or adoptive immunotherapy refers to the adoptive transfer of human T lymphocytes that express antigen receptors that are specific for target cells. For example, human T lymphocytes may express TCRs that are specific for peptide MHC complexes expressed on target cells or chimeric antigen receptors (CAR) that are specific for antigens expressed on target cells..

This can be used to treat a range of diseases depending upon the target chosen, e.g., tumour specific antigens to treat cancer. Adoptive cellular therapy involves removing a portion of a donor's or the patient's cells, for example, white blood cells. The cells are then used to generate iPSCs *in vitro* and these iPSCs are used to efficiently generate T cells specific for peptide MHC complexes on target cells as described herein. The T cells may be expanded, washed, concentrated, and/or then frozen to allow time for testing, shipping and storage until a patient is ready to receive the infusion of cells.

Other aspects of the invention provide the use of a population of T cells as described herein for the manufacture of a medicament for the treatment of cancer, a population of T cells as described herein for the treatment of cancer, and a method of treatment of cancer comprising administering a population of T cells as described herein to an individual in need thereof.

The population of T cells may be autologous i.e. the T cells are produced from iPSCs derived from cells originally obtained from the same individual to whom the T cells are subsequently administered (i.e. the donor and recipient individual are the same).

The population of T cells may be allogeneic i.e. the T cells may be produced from iPSCs derived from cells originally obtained from a different individual to the individual to whom the T cells are subsequently administered (i.e. the donor and recipient individual are different). Allogeneic refers to a graft derived from a different animal of the same species.

The donor and recipient individuals may be HLA matched to avoid GVHD and other undesirable immune effects, such as rejection. Alternatively, the donor and recipient individuals may not be HLA matched, or HLA genes in the cells from the donor individual may be modified, for example by gene editing, to remove any HLA mismatch with the recipient.

A suitable population of T cells for administration to a recipient individual may be produced by a method comprising providing an initial population of cells obtained from a donor individual, reprogramming the cells into iPSCs, inactivating RAG in the iPSCs and differentiating the RAG inactivated iPSCs into T cells that express an antigen receptor, such as an αβ TCR which binds specifically to cancer cells in the recipient individual, as described herein.

Following administration of the T cells, the recipient individual may exhibit a T cell mediated immune response against cancer cells in the recipient individual. This may have a beneficial effect on the cancer condition in the individual.

As used herein, the terms "cancer," "neoplasm," and "tumour" are used interchangeably and, in either the singular or plural form, refer to cells that have undergone a malignant transformation that makes them pathological to the host organism.

Primary cancer cells can be readily distinguished from non-cancerous cells by well-established techniques, particularly histological examination. The definition of a cancer cell, as used herein, includes not only a primary cancer cell, but any cell derived from a cancer cell ancestor. This includes metastasized cancer cells, and in vitro cultures and cell lines derived from cancer cells. When referring to a type of cancer that normally manifests as a solid tumour, a "clinically detectable" tumour is one that is detectable on the basis of tumour mass; e.g., by procedures such as computed tomography (CT) scan, magnetic resonance imaging (MRI), X-ray, ultrasound or palpation on physical examination, and/or which is detectable because of the expression of one or more cancer-specific antigens in a sample obtainable from a patient.

Cancer conditions may be characterised by the abnormal proliferation of malignant cancer cells and may include leukaemias, such as AML, CML, ALL and CLL, lymphomas, such as Hodgkin lymphoma, non-Hodgkin lymphoma and multiple myeloma, and solid cancers such as sarcomas, skin cancer, melanoma, bladder cancer, brain cancer, breast cancer, uterus cancer, ovary cancer, prostate cancer, lung cancer, colorectal cancer, cervical cancer, liver cancer, head and neck cancer, oesophageal cancer, pancreas cancer, renal cancer, adrenal cancer, stomach cancer, testicular cancer, cancer of the gall bladder and biliary tracts, thyroid cancer, thymus cancer, cancer of bone, and cerebral cancer, as well as cancer of unknown primary (CUP).

Cancer cells within an individual may be immunologically distinct from normal somatic cells in the individual (i.e. the cancerous tumour may be immunogenic). For example, the cancer cells may be capable of eliciting a systemic immune response in the individual against one or more antigens expressed by the cancer cells. The tumour antigens that elicit the immune response may be specific to cancer cells or may be shared by one or more normal cells in the individual.

The cancer cells of an individual suitable for treatment as described herein may express the antigen and may be of correct HLA type to bind the heterologous TCR expressed by the T cells.

An individual suitable for treatment as described above may be a mammal. In preferred embodiments, the individual is a human. In other preferred embodiments, non-human mammals, especially mammals that are conventionally used as models for demonstrating therapeutic efficacy in humans (*e.g*. murine, primate, porcine, canine, or rabbit animals) may be employed.

In some embodiments, the individual may have minimal residual disease (MRD) after an initial cancer treatment.

An individual with cancer may display at least one identifiable sign, symptom, or laboratory finding that is sufficient to make a diagnosis of cancer in accordance with clinical standards known in the art. Examples of such clinical standards can be found in textbooks of medicine such as Harrison's Principles of Internal Medicine, 15th Ed., Fauci AS et al., eds., McGraw-Hill, New York, 2001. In some instances, a diagnosis of a cancer in an individual may include identification of a particular cell type (e.g. a cancer cell) in a sample of a body fluid or tissue obtained from the individual.

An anti-tumour effect is a biological effect which can be manifested by a reduction in the rate of tumour growth, decrease in tumour volume, a decrease in the number of tumour cells, a decrease in the number of metastases, an increase in life expectancy, or amelioration of various physiological symptoms associated with the cancerous condition. An "anti-tumour effect" can also be manifested by the ability of the peptides, polynucleotides, cells and antibodies described herein in prevention of the occurrence of tumour in the first place

Treatment may be any treatment and therapy, whether of a human or an animal (e.g. in veterinary applications), in which some desired therapeutic effect is achieved, for example, the inhibition or delay of the progress of the condition, and includes a reduction in the rate of progress, a halt in the rate of progress, amelioration of the condition, cure or remission (whether partial or total) of the condition, preventing, delaying, abating or arresting one or more symptoms and/or signs of the condition or prolonging survival of a subject or patient beyond that expected in the absence of treatment.

Treatment may also be prophylactic (i.e. prophylaxis). For example, an individual susceptible to or at risk of the occurrence or re-occurrence of cancer may be treated as described herein. Such treatment may prevent or delay the occurrence or re-occurrence of cancer in the individual.

In particular, treatment may include inhibiting cancer growth, including complete cancer remission, and/or inhibiting cancer metastasis. Cancer growth generally refers to any one of a number of indices that indicate change within the cancer to a more developed form. Thus, indices for measuring an inhibition of cancer growth include a decrease in cancer cell survival, a decrease in tumour volume or morphology (for example, as determined using computed tomographic (CT), sonography, or other imaging method), a delayed tumour growth, a destruction of tumour vasculature, improved performance in delayed hypersensitivity skin test, an increase in the activity of T cells, and a decrease in levels of tumour-specific antigens. Administration of T cells modified as described herein may improve the capacity of the individual to resist cancer growth, in particular growth of a cancer already present in the subject and/or decrease the propensity for cancer growth in the individual.

The T cells or the pharmaceutical composition comprising the T cells may be administered to a subject by any convenient route of administration, whether systemically/ peripherally or at the site of desired action, including but not limited to; parenteral, for example, by infusion. Infusion involves the administration of the T cells in a suitable composition through a needle or catheter. Typically, T cells are infused intravenously or subcutaneously, although the T cells may be infused via other non-oral routes, such as intramuscular injections and epidural routes. Suitable infusion techniques are known in the art and commonly used in therapy (see, e.g., Rosenberg et al., New Eng. J. of Med., 319:1676, 1988).

Typically, the number of cells administered is from about 10⁵ to about 10¹⁰ per Kg body weight, typically 2×10⁸ to 2×10¹⁰ cells per individual, typically over the course of 30 minutes, with treatment repeated as necessary, for example at intervals of days to weeks. It will be appreciated that appropriate dosages of the TCR αβ+ T cells, and compositions comprising the TCR αβ+ T cells, can vary from patient to patient. Determining the optimal dosage will generally involve the balancing of the level of therapeutic benefit against any risk or deleterious side effects of the treatments of the present invention. The selected dosage level will depend on a variety of factors including, but not limited to, the activity of the particular cells, cytokine release syndrome (CRS), the route of administration, the time of administration, the rate of loss or inactivation of the cells, the duration of the treatment, other drugs, compounds, and/or materials used in combination, and the age, sex, weight, condition, general health, and prior medical history of the patient. The amount of cells and the route of administration will ultimately be at the discretion of the physician, although generally the dosage will be to achieve local concentrations at the site of action which achieve the desired effect without causing substantial harmful or deleterious side-effects.

While the T cells may be administered alone, in some circumstances the T cells may be administered in combination with the target antigen, APCs displaying the target antigen, CD3/CD28 beads, IL-2, IL-7 and/or IL15 to promote expansion *in vivo* of the population of T cells.

The population of T cells may be administered in combination with one or more other therapies, such as cytokines e.g. IL-2, CD4+ CD8+ chemotherapy, radiation and immuno-oncology agents, including checkpoint inhibitors, such as anti-B7-H3, anti-B7-H4, anti-TIM3, anti-KIR, anti-LAG3, anti-PD-1, anti-PD-L1, and anti-CTLA4 antibodies.

The one or more other therapies may be administered by any convenient means, preferably at a site which is separate from the site of administration of the T cells.

Administration of T cells can be effected in one dose, continuously or intermittently (e.g., in divided doses at appropriate intervals) throughout the course of treatment. Methods of determining the most effective means and dosage of administration are well known to those of skill in the art and will vary with the formulation used for therapy, the purpose of the therapy, the target cell being treated, and the subject being treated. Single or multiple administrations can be carried out with the dose level and pattern being selected by the treating physician. Preferably, the T cells are administered in a single transfusion of a least 1 × 10⁹ T cells.

Other aspects and embodiments of the invention provide the aspects and embodiments described above with the term "comprising" replaced by the term "consisting of" and the aspects and embodiments described above with the term "comprising" replaced by the term "consisting essentially of'.

It is to be understood that the application discloses all combinations of any of the above aspects and embodiments described above with each other, unless the context demands otherwise. Similarly, the application discloses all combinations of the preferred and/or optional features either singly or together with any of the other aspects, unless the context demands otherwise.

Modifications of the above embodiments, further embodiments and modifications thereof will be apparent to the skilled person on reading this disclosure, and as such, these are within the scope of the present invention.

All documents and sequence database entries mentioned in this specification are incorporated herein by reference in their entirety for all purposes.
"and/or" where used herein is to be taken as specific disclosure of each of the two specified features or components with or without the other. For example "A and/or B" is to be taken as specific disclosure of each of (i) A, (ii) B and (iii) A and B, just as if each is set out individually herein.

### Experimental

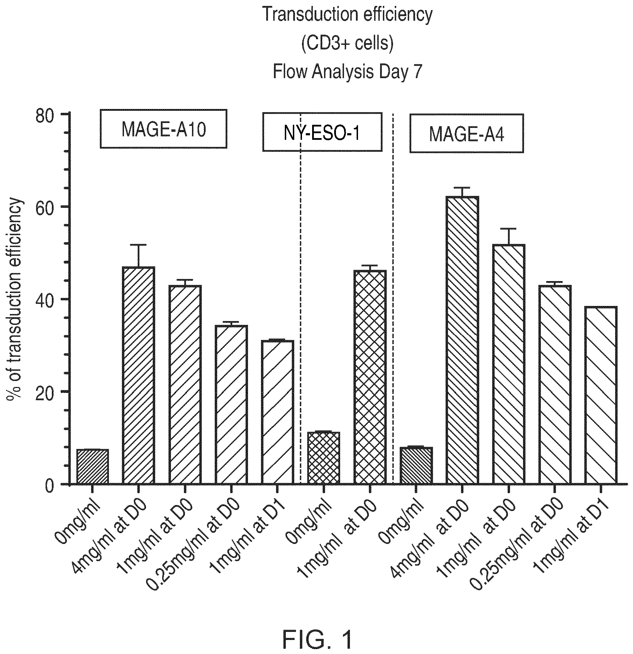

T-cells were exposed to 0.25mg/ml, 1mg/ml or 4mg/ml poloxamer at day 0 or day 1 and then transduced with a lentiviral vector encoding a MAGE-A10, NY-ESO-1 or MAGE-A4 TCR. Transduction efficiency was determined using flow analysis to identify the proportion of CD3+ cells in the population. For all TCRs, the presence of poloxamer was shown to increase transduction efficiency (Figure 1). In addition, exposure with poloxamer at day 0 was found to improve transduction efficiency relative to exposure to poloxamer at day 1 (Figure 1). The efficiency of transduction was also found to increase with increasing concentration of poloxamer (Figure 1).

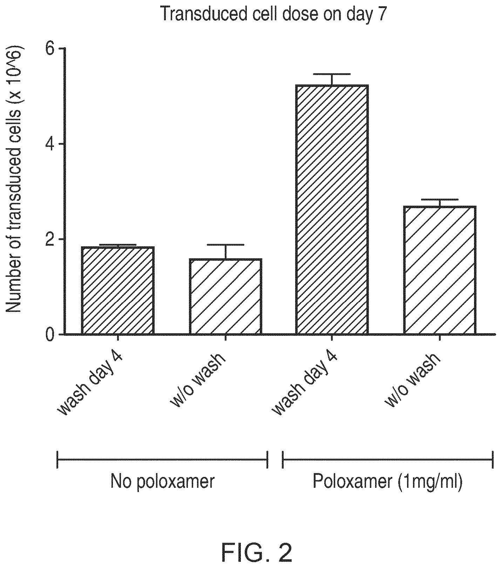

The number of transduced T-cells was determined at day 7 following transduction at day 1 by lentivirus in presence or absence of 1 mg/ml poloxamer F108 with or without cell washing on day 4. Cell washing on day 4 was found to be required for optimal cell expansion (Figure 2).

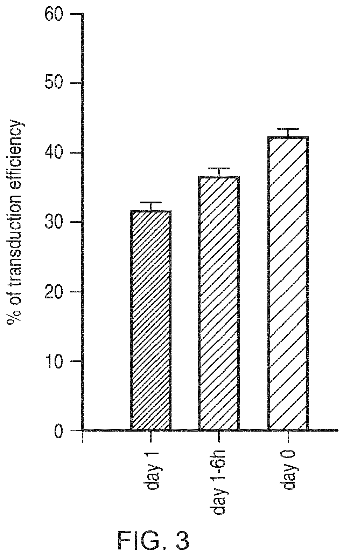

The effect on efficiency of lentiviral transduction of exposing T cells to poloxamer F108 was determined. Transduction efficiency was found to be increased at day 1 - 6 hours (day 0 + 18 hours) exposure and further increased at day 0 exposure relative to day 1 exposure (Figure 3).

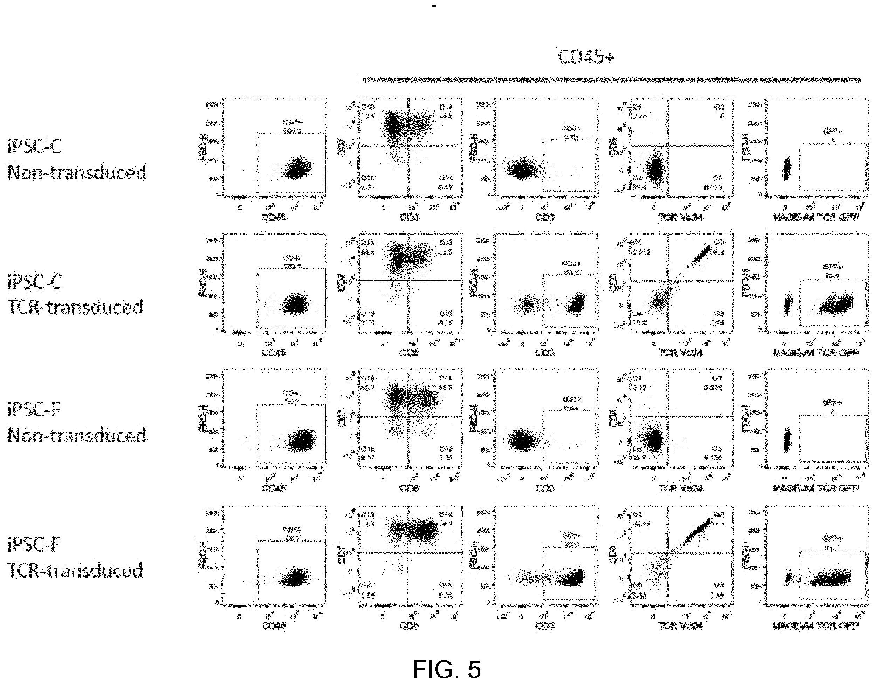

The efficiency of lentiviral transduction of exposing CD34+ hemogenic precursor cells differentiated from hiPSC was investigated. TCRs specific to an HLA-A2 restricted peptide from MAGE-A4 were found to be efficiently expressed in T-cells differentiated from the CD34+ haemogenic precursor cells (Figure 5). Active TCRs were also found to be efficiently expressed in regulatory T-cells prepared according to the methods set out in WO/2018/185166.

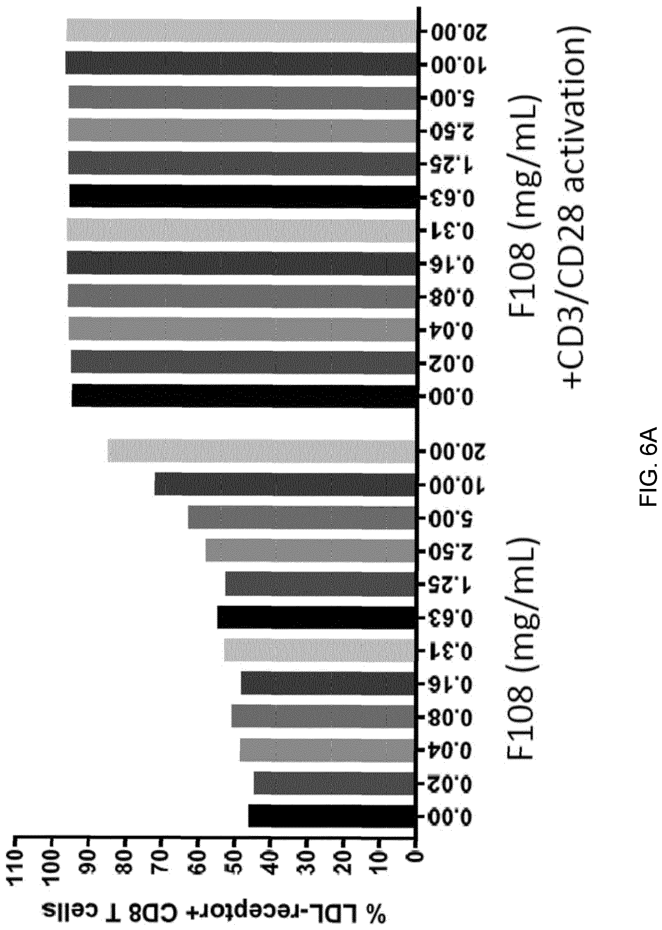

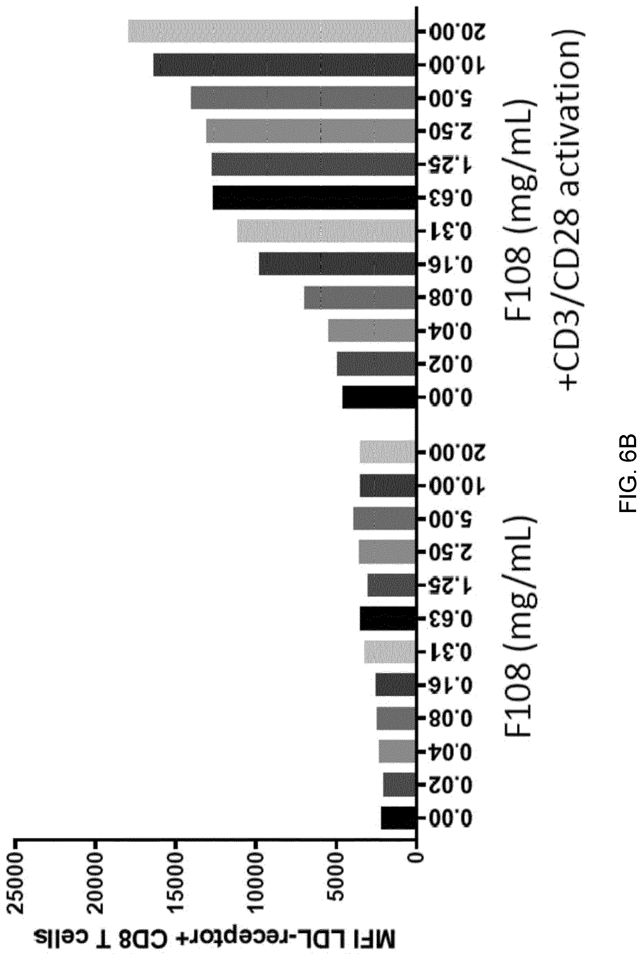

Poloxamer F108 was found to cause an increase in cell surface low density lipoprotein receptor (LDL-R) (Figure 6A and 6B). When combined with CD3/CD28 activation, poloxamer F108 did not affect the number of cells expressing LDL-R. However, poloxamer F108 was observed to increase the amount of LDL-R per cell, measured by mean fluorescence intensity (MFI).

Whilst not wishing to be bound by theory, LDL-R is the major binding site for VSV-G pseudotyped viruses (Amirache et al Blood 2014:123 1422-1424) and the preincubation time allows for enhanced cell surface expression of this receptor resulting in the increase in transduction. This phenomenon of increased surface LDL-R is surprising as poloxamer has been reported to down-regulate LDL-R in vivo (Leon et al, Pharm Res 2006 23(7), 1597-1607).

## Claims

1. A method of transducing mammalian cells comprising:
(i) exposing a population of mammalian cells to a poloxamer in the absence of a lentiviral vector for 6 hours or more to produce a transduction-primed mammalian cell population and
(ii) exposing the transduction-primed mammalian cell population to a lentiviral vector, such that the T cells are transduced with the lentiviral vector; and
(iii) separating the transduced mammalian cells from the poloxamer.

2. A method according to claim 1 wherein the population of mammalian cells is exposed to the poloxamer in the absence of the lentiviral vector for 12 hours or more to produce the transduction-primed mammalian cell population.

3. A method according to claim 2 wherein the population of mammalian cells is exposed to the poloxamer in the absence of a lentiviral vector for about 24 hours to produce the transduction-primed mammalian cell population.

4. A method according to any one of the preceding claims wherein the transduced mammalian cells are separated from the poloxamer after 48 to 96 hours of exposure to the lentiviral vector.

5. A method according to claim 4 wherein the transduced mammalian cells are separated from the poloxamer after about 72 hours of exposure to the lentiviral vector.

6. A method according to any one of the preceding claims wherein the poloxamer has an average molecular weight of 10.0kDa to 15 kDa

7. A method according to claim 6 wherein the poloxamer is poloxamer 407 or poloxamer 338.

8. A method according to any one of the preceding claims wherein the mammalian cell population is exposed to the poloxamer by culturing the mammalian cells in a priming medium comprising the poloxamer.

9. A method according to claim 8 wherein the priming medium comprises 10 µg/ml to 100mg/ml poloxamer.

10. A method according to any one of the preceding claims wherein the lentiviral vector comprises a nucleic acid encoding a heterologous antigen receptor.

11. A method according to claim 10 wherein the transduced mammalian cells express the heterologous antigen receptor.

12. A method according to claim 10 or claim 11 wherein the heterologous antigen receptor is a chimeric antigen receptor (CAR).

13. A method according to claim 10 or claim 11 wherein the heterologous antigen receptor is a T cell receptor (TCR).

14. A method according to claim 13 wherein the heterologous TCR is HLA-A*02-restricted

15. A method according to claim 13 or claim 14 wherein the heterologous TCR is an affinity enhanced TCR.

16. A method according to any one of claims 10 to 15 wherein the heterologous antigen receptor binds to a tumour antigen or tumour associated antigen.

17. A method according to claim 16 wherein the tumour antigen is alpha-fetoprotein (AFP), NY-ESO1, MAGE-A10 or MAGE-A4.

18. A method according to any one of the preceding claims wherein the mammalian cell population is exposed to the lentiviral vector by culturing the mammalian cells in a transduction medium comprising the lentiviral vector.

19. A method according to any one of the preceding claims wherein the transduction medium further comprises the poloxamer.

20. A method according to any one of the preceding claims wherein the mammalian cells are cultured in the transduction medium for 1 to 4 days.

21. A method according to any one of the preceding claims comprising isolating or purifying the transduced mammalian cell population.

22. A method according to claim 21 wherein transduced mammalian cell population isolated from the population by fluorescence-activated cell sorting.

23. A method according to any one of the preceding claims comprising expanding the population of transduced mammalian cells.

24. A method according to according to any one of the preceding claims comprising concentrating the population of transduced mammalian cells.

25. A method according to according to any one of the preceding claims comprising storing the population of transduced mammalian cells.

26. A method according to any one of the preceding claims comprising formulating the population of transduced mammalian cells with a pharmaceutically acceptable excipient.

27. A method according to any one of the preceding claims wherein the mammalian cells are T cells.

28. A method according to any one of claims 1 to 26 wherein the mammalian cells are progenitor cells capable of differentiation into T cells.

29. A method according to claim 28 wherein the progenitor cells are iPSCs, mesoderm cells, haemogenic endothelial cells, haematopoietic progenitor cells or progenitor T cells.
